# FASCICULE DE BREVET EUROPEEN

(11) **EP 4 248 854 B1**
(45) Date de publication et mention de la délivrance du brevet: **29.05.2024**
(21) Numéro de dépôt: 22214369.5
(22) Date de dépôt: 16.12.2022
(51) Int. Cl.: A61B 5/053, G01G 19/50

(54) **STATION DE MESURE AVEC POIGNÉE**
MESSSTATION MIT GRIFF
MEASURING STATION WITH GRIP

(30) Priorité: 31.12.2021 FR 2114742
(43) Date de publication de la demande: 27.09.2023
(73) Titulaire: Withings, 92130 Issy-les-Moulineaux (FR)
(72) Inventeur: Wang, Jean-Louis, 92130 Issy-les-Moulineaux (FR); Richard, Arthur, 92130 Issy-les-Moulineaux (FR); Merlot, Antoine, 92130 Issy-les-Moulineaux (FR); Callens, Victorine, 92130 Issy-les-Moulineaux (FR); Geffraye, Victor, 92130 Issy-les-Moulineaux (FR); Droze, Thomas, 92130 Issy-les-Moulineaux (FR)
(74) Mandataire: Withings IP

(56) Documents cités:
- EP-A1- 1 700 565
- CN-A- 112 022 102
- CN-U- 204 909 451
- CN-U- 211 534 389
- JP-A- 2003 299 628
- JP-A- 2010 057 540

## Description

### Domaine technique

. La présente description concerne la surveillance de la santé d'un utilisateur et plus spécifiquement concerne les stations de mesure permettant de mettre en oeuvre une ou plusieurs mesures de signaux biométriques (ou paramètres physiologiques) d'un utilisateur.
Au moins une des données suivantes est déterminée par la station de mesure de la présente demande : poids ou masse, électrocardiogramme (ECG), impédancemétrie (analyse d'impédance du corps humain), dont impédance-pléthysmogramme (IPG) impedance-cardiogramme (ICG) et bioimpédance (« *bioimpedance analysis* » BIA, pour la masse de graisse, d'eau, de muscles, etc.), photoplethysmogramme (PPG), ballistocardiogramme (BCG), analyse de la conductance électrochimique de la peau (« ESC analysis » pour « *electrochemical skin conductance* » ou plus simplement « ESC » dans la présente description) et évaluation de la fonction sudorale (parfois appelée « *sudogramme* » dans la présente demande), rythme cardiaque (« *heart rate* », HR), vitesse d'onde de pouls vitesse d'onde de pouls (« *pulse wave velocity* », PWV), etc.

### Etat de la technique

. Le document WO2010/122252, de 2010, décrit une balance connectée avec mesure de poids et de bioimpédance. Les documents EP3087914 et EP3095380, de 2015, décrivent quant à eux une balance connectée permettant d'obtenir des informations sur l'état cardiovasculaire de l'utilisateur, avec notamment une mesure de PTT (« *pulse transit time* ») à l'aide d'un BCG et d'un IPG.

. Le document CN211534389 décrit une balance avec poignée pour faire de la bioimpédance segmentale. Le document WO2021/164561 décrit une balance avec poignée pour faire un ECG. Les documents CN 112 022 102, EP 1 700 565, JP 2010 057540, CN 211 534 389 et JP 2003 299628 décrivent des balances avec une poignée.

### Présentation de l'invention

. La présente description vise à proposer une station de mesure permettant l'obtention de différentes mesures de signaux biométriques, avec une qualité accrue.

. L'invention est définie dans les revendications.

. Dans un mode de réalisation, la présente description présente une station de mesure comprenant :
- une base comprenant :
   ∘ une plaque de mesure présentant une face supérieure apte à recevoir les pieds d'un utilisateur,
   ∘ un support de poignée, monté sur la face supérieure de la plaque de mesure,
- une poignée apte à recevoir les mains d'un utilisateur, la poignée étant configurée pour être reçue sur le support de poignée.

. En particulier, le support de poignée peut avoir une portion qui définit un berceau. Le berceau peut avoir une forme complémentaire à celle de la poignée.

. Dans un mode de réalisation, la station de mesure comprend un câble reliant la poignée à la base. Le câble comprend un câblage électrique pour faire transiter des signaux électriques.

. Dans un mode de réalisation, la plaque de mesure comprend un orifice traversant au travers duquel passe le câble reliant la poignée à la base. L'orifice traversant peut être cylindrique et allongé, en particulier selon une direction orthogonale à la plaque de mesure.

. Dans un mode de réalisation, le support de poignée comprend un orifice traversant au travers duquel passe le câble. L'orifice traversant peut être cylindrique et allongé, en particulier selon une direction orthogonale à la plaque de mesure.

. Dans un mode de réalisation, les deux orifices traversants sont en regard l'un de l'autre.

. Dans un mode de réalisation, le câble s'étend orthogonalement depuis une surface de la poignée, afin de pouvoir s'insérer dans les orifices traversants lorsque la poignée est positionnée dans le support de poignée.

. Dans un mode de réalisation, l'orifice traversant présente un chanfrein pour supprimer l'arrête vive.

. Dans un mode de réalisation, la base comprend un enrouleur apte à enrouler et dérouler le câble.

. Dans un mode de réalisation, la base comprend un rouleau configuré pour faciliter un changement de direction du câble lors de l'enroulement et du déroulement du câble.

. Dans un mode de réalisation, la station de meure comprend un deuxième rouleau configuré pour canaliser le déplacement du câble, les deux rouleaux étant de part et d'autre du câble.

. Dans un mode de réalisation, la station comprend une plaque de support disposée à l'intérieur de la base et sur laquelle est monté le rouleau.

. Dans un mode de réalisation, en position rangée, le câble est dissimulé par la poignée et le support de poignée.

. Le support de poignée forme un berceau configuré pour recevoir la poignée, notamment un berceau de forme complémentaire à celle de la poignée.

. Dans un mode de réalisation, l'orifice traversant du support de poignée est positionné au fond du berceau.

. Dans un mode de réalisation, le support de poignée s'étend selon une direction transversale le long de la largeur de la base.

. Le berceau s'étend sur une surface, dans une section transversale du support de poignée, de moins de 90° de part et d'autre à partir d'un fond du berceau.

. Dans un mode de réalisation, la station de mesure comprend une attache magnétique dans la poignée et le support de poignée, afin de contribuer à maintenir la poignée dans le support de poignée.

. Dans un mode de réalisation, la poignée s'étend entre deux extrémités et comprend une pluralité d'électrodes disposées à la suite les unes des autres entre les deux extrémités. La poignée peut s'étendre selon une direction longitudinale ou avoir une forme courbée. Les électrodes sont espacées entre elles pour les isoler les unes des autres.

. Dans un mode de réalisation, les électrodes s'étendent, dans une section transversale, au plus sur une surface externe de la poignée comprise entre -45° et +90°, 0° étant défini par une position d'insertion du câble dans la poignée, la plage entre 0° et +180° étant définie comme tournée vers un bord antérieur de la station de mesure.

. Dans un mode de réalisation, le berceau s'étend sur une surface dont l'angle dans une section transversale du support de poignée, est au moins égal à celui des électrodes dans le sens des angles négatifs.

. Dans un mode de réalisation, la plaque de mesure est plane.

. Dans un mode de réalisation, la plaque de mesure est en verre.

. Dans un mode de réalisation, la poignée est configurée pour permettre l'acquisition d'un électrocardiogramme (ECG) et une analyse d'impédance (BIA, IPG).

. La présente description présente aussi, dans un mode de réalisation, une station de mesure comprenant :
une base comprenant une plaque de mesure présentant une face supérieure apte à recevoir les pieds d'un utilisateur,
une poignée apte à recevoir les mains d'un utilisateur, la poignée s'étendant entre deux extrémités et comprenant une pluralité d'électrodes disposées à la suite les unes des autres entre les deux extrémités, notamment avec un espacement entre elles.

. La station de mesure peut comprendre un câble reliant la poignée à la base,

. Dans un mode de réalisation, les électrodes s'étendent, dans une section transversale, au plus sur une surface externe de la poignée comprise entre -45° et +90°, 0° étant définie par une position d'insertion du câble dans la poignée, la plage entre 0 et +180° étant définie comme tournée vers un bord antérieur de la station de mesure, la plage entre 0 et -180° étant définie comme tournée vers un bord postérieur de la station de mesure (lorsque la poignée est en position rangée).

. Dans un mode de réalisation, dans une section transversale de la poignée qui inclut une électrode, l'angle défini par le barycentre de la surface externe et l'extrémité des électrodes est compris entre -45° et +90° (notamment -30° et +80°), avec 0° qui est l'angle défini par le point de la surface externe qui est le plus proche de la plaque de mesure lorsque la poignée est en position rangée, les angles positifs (0° à +180°) étant définis à partir de 0°en allant du côté du bord antérieur et les angles négatifs (0° à -180°) étant définis à partir de 0° en allant du côté du bord postérieur. Dans une section transversale incluant le câble, le point d'entrée du câble est à 0°.

. La présente description présente aussi, dans un mode de réalisation, une station de mesure comprenant :
une base comprenant une plaque de mesure présentant une face supérieure apte à recevoir les pieds d'un utilisateur,
une poignée apte à recevoir les mains d'un utilisateur, la poignée comprenant une pluralité d'électrodes

. Dans un mode de réalisation, chaque électrode s'étend, dans une section transversale, au plus sur une surface externe de la poignée comprise entre -45° et +90°, 0° étant définie par la position d'insertion d'un câble dans la poignée, la plage entre 0° et +180° étant définie comme tournée vers un bord antérieur de la station de mesure, la plage entre 0° et -180° étant définie comme tournée vers un bord postérieur de la station de mesure (lorsque la poignée est en position rangée dans un support de poignée).

. Dans un mode de réalisation, dans une section transversale de la poignée qui inclut une électrode, l'angle défini par le barycentre de la surface externe et l'extrémité des électrodes est compris entre -45° et +90° (notamment -30° et +80°), avec 0° qui est l'angle défini par le point de la surface externe qui est le plus proche de la plaque de mesure lorsque la poignée est en position rangée, les angles positifs (0° à +180°) étant définis à partir de 0°en allant du côté du bord antérieur et les angles négatifs (0° à -180°) étant définis à partir de 0° en allant du côté du bord postérieur.

. Dans une mode de réalisation, la station de mesure comprend un câble reliant la poignée à la base ; dans une section transversale de la poignée incluant le point d'entrée du câble dans la poignée, ledit point d'entrée du câble est à 0°

. Dans un mode de réalisation, la station de mesure comprend un support de poignée configuré pour recevoir la poignée. En particulier, le support de poignée peut avoir une portion qui définit un berceau. Le berceau peut avoir une forme complémentaire à celle de la poignée.

. Dans un mode de réalisation, le berceau s'étend sur une surface dont l'angle dans une section transversale, est au moins égal à celui des électrodes dans le sens des angles négatifs, lorsque la poignée est en position rangée sur le support de poignée.

. La station de mesure peut comprendre les autres caractéristiques décrites précédemment.

### Description des figures

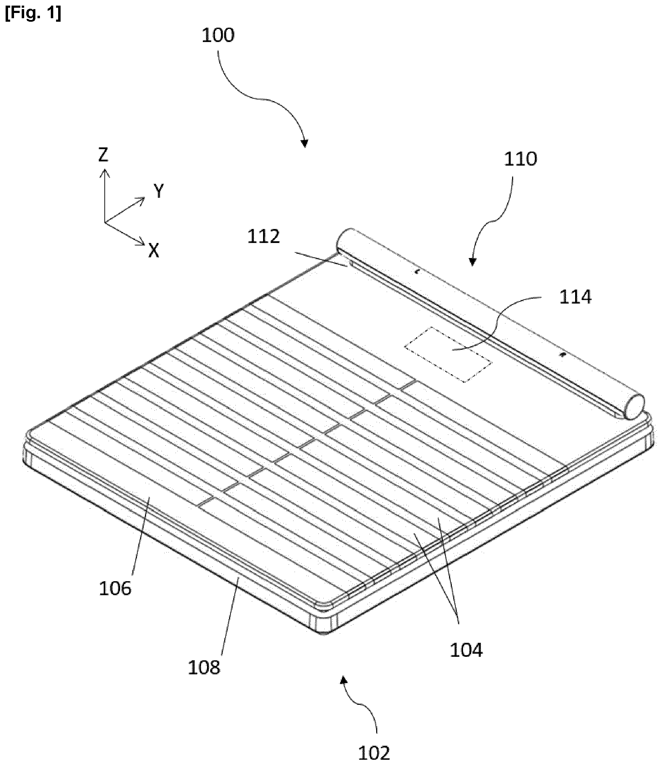

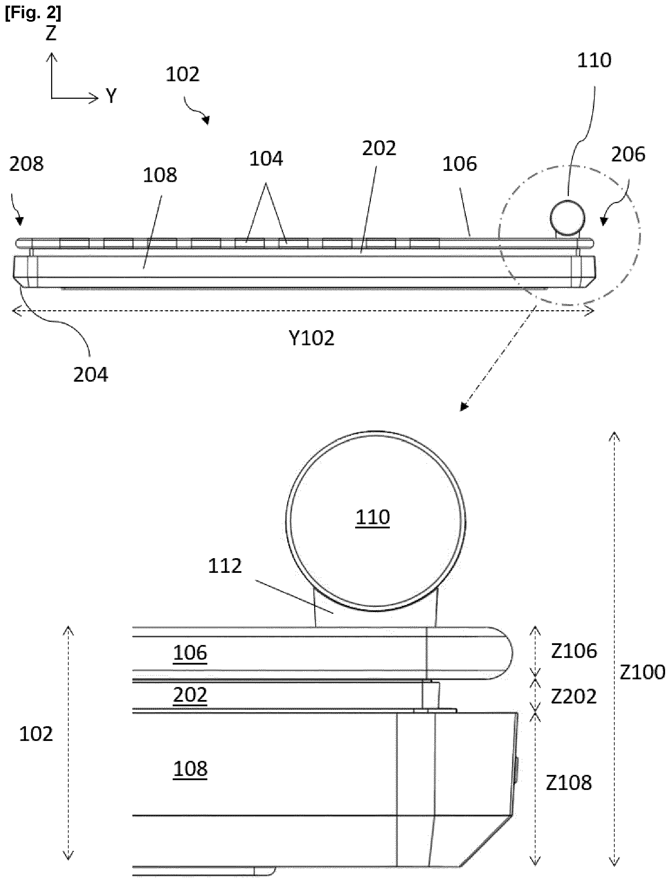

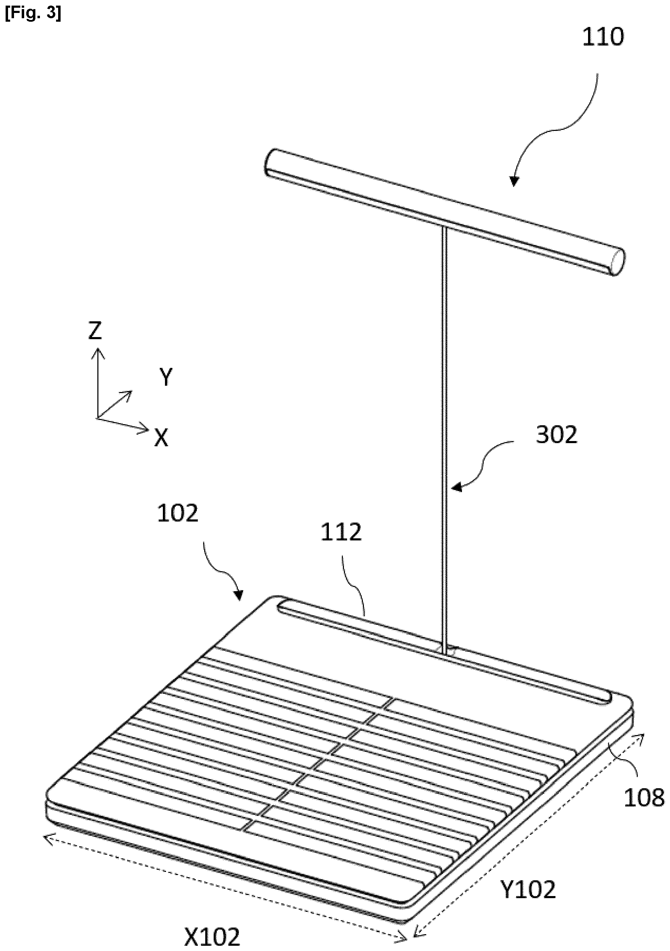

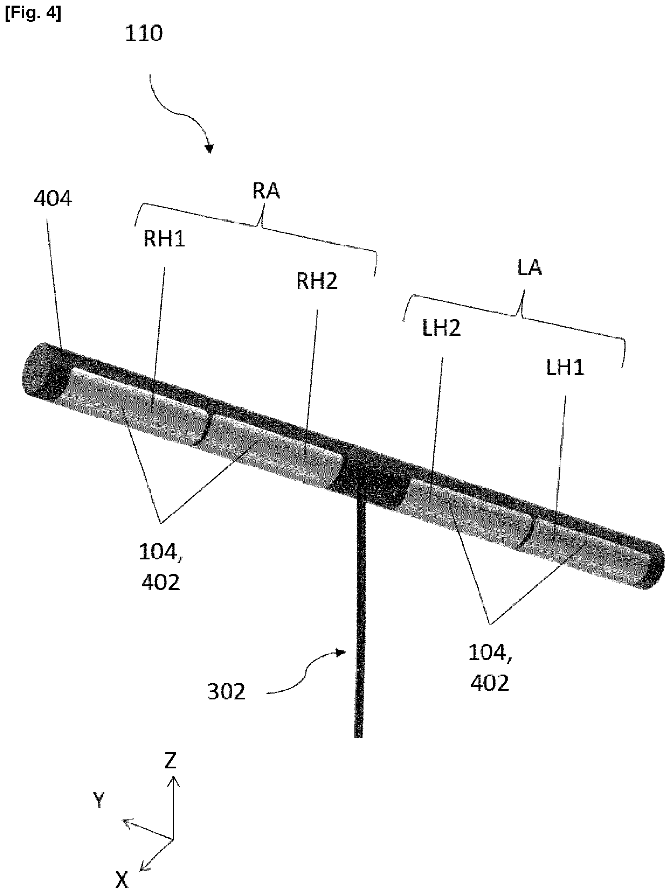

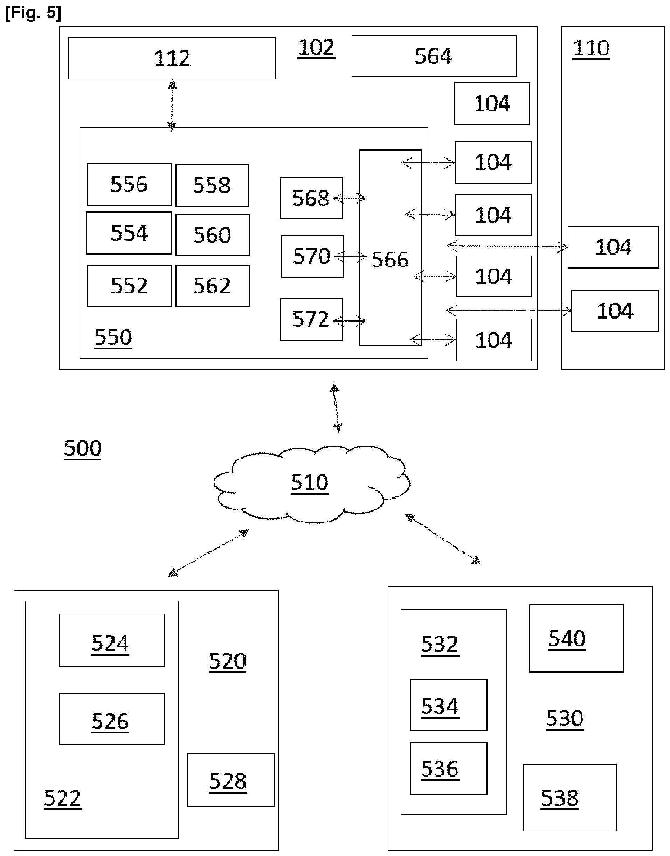

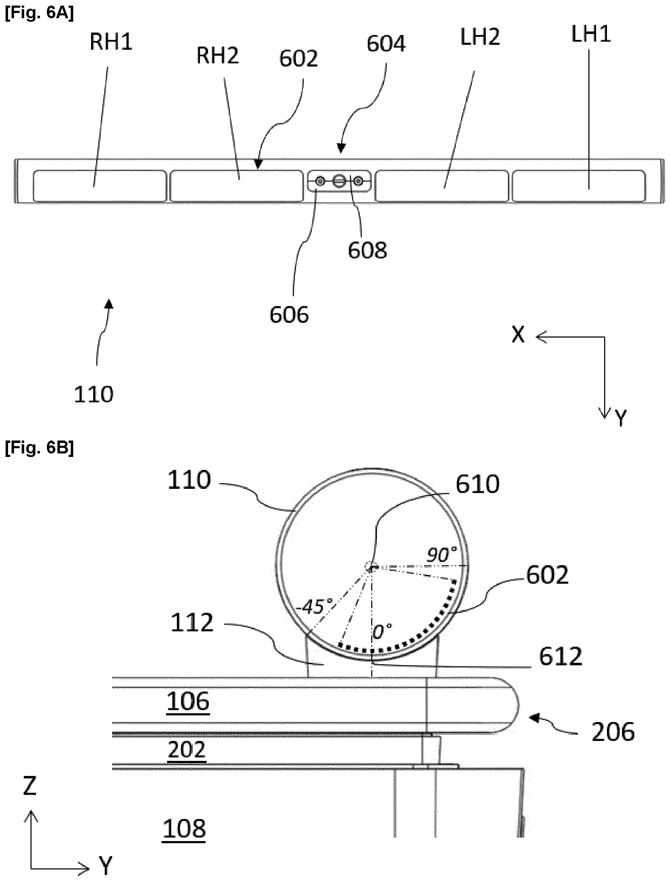

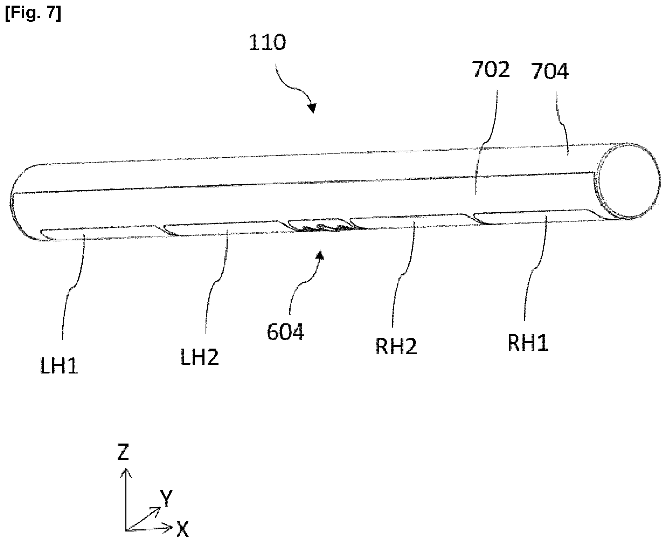

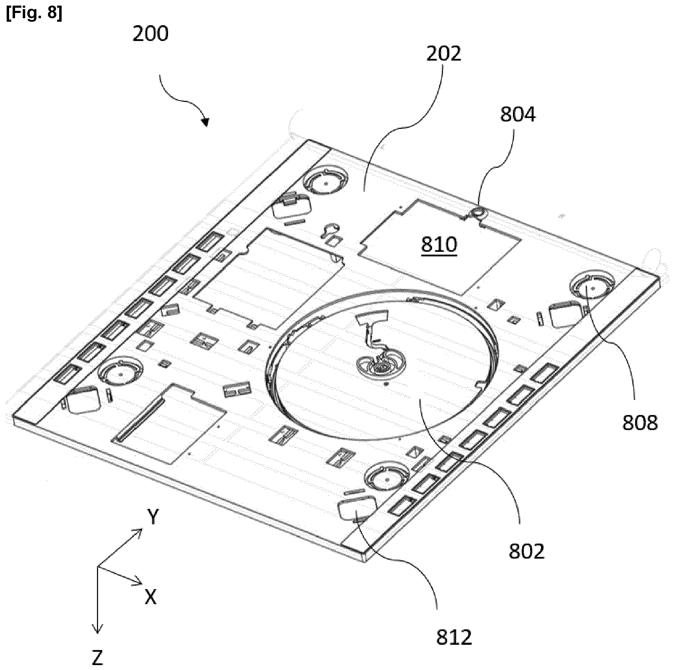

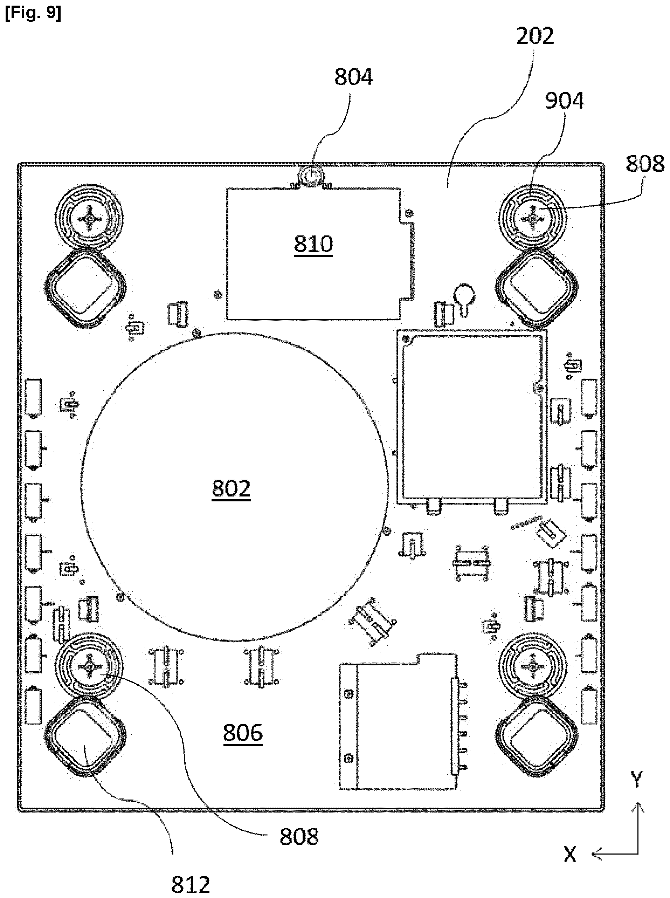

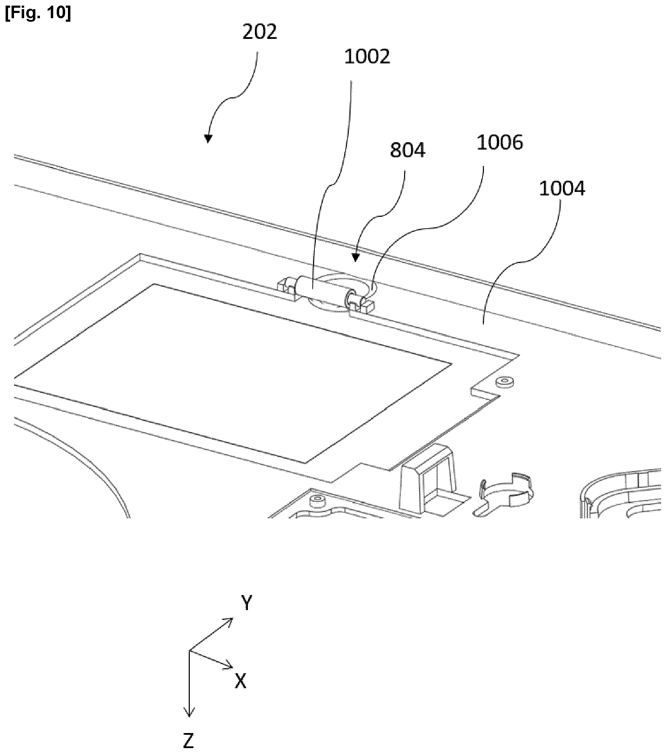

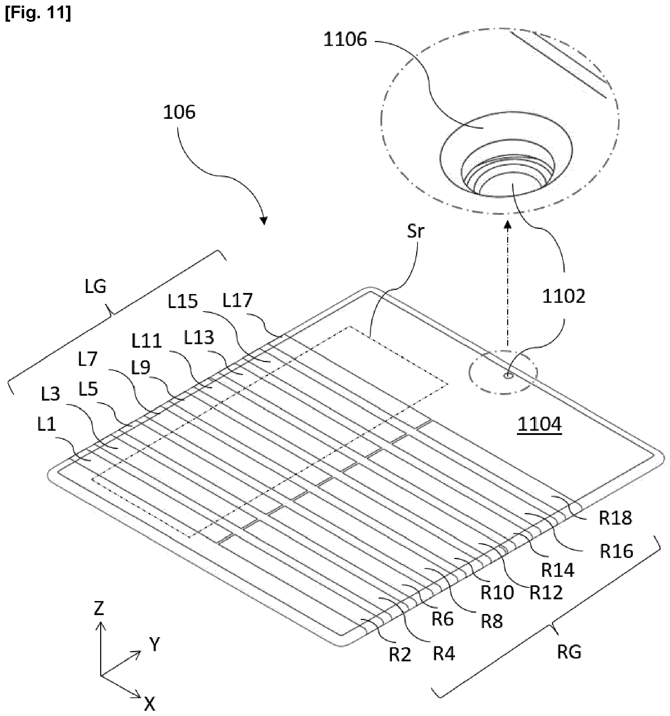

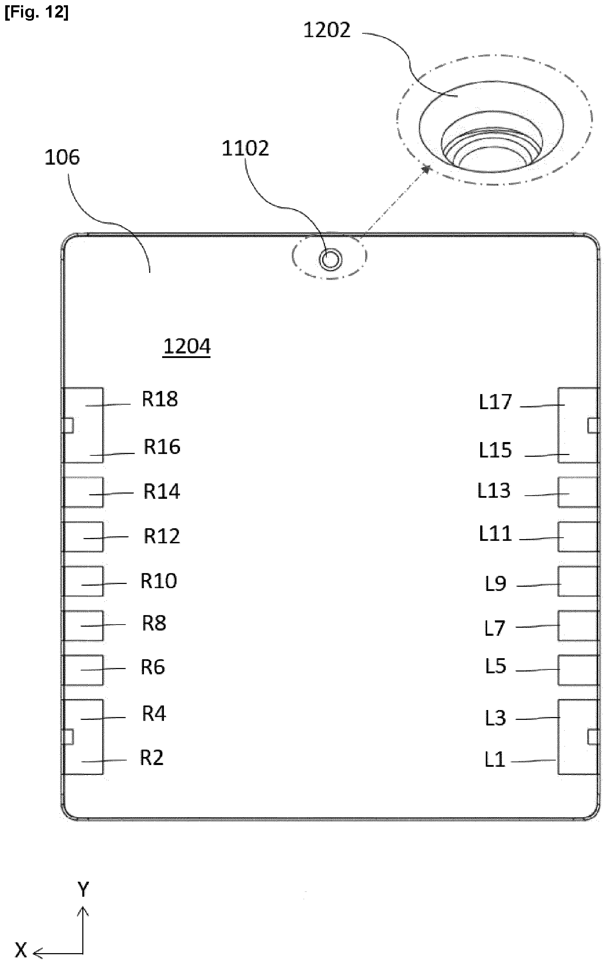

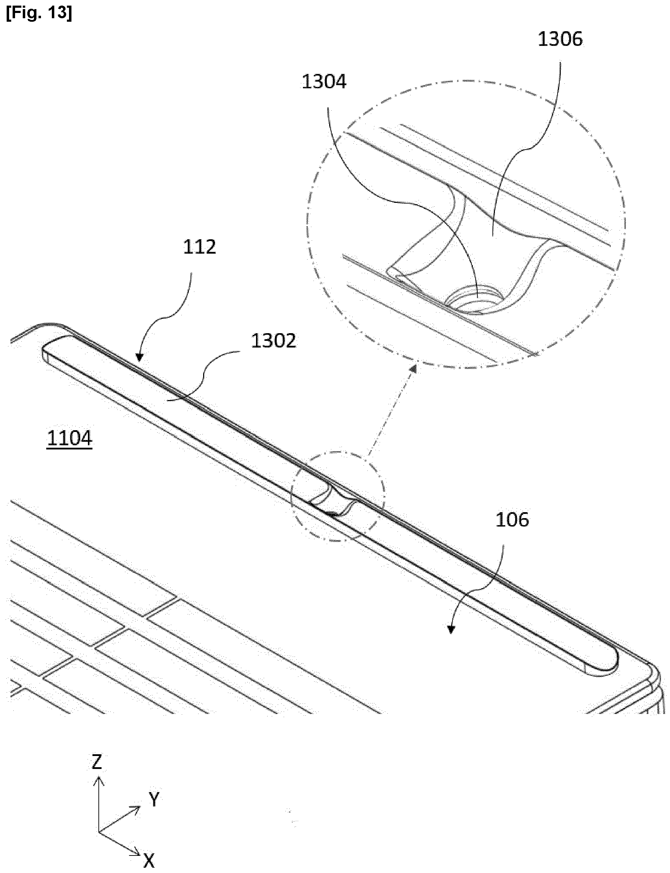

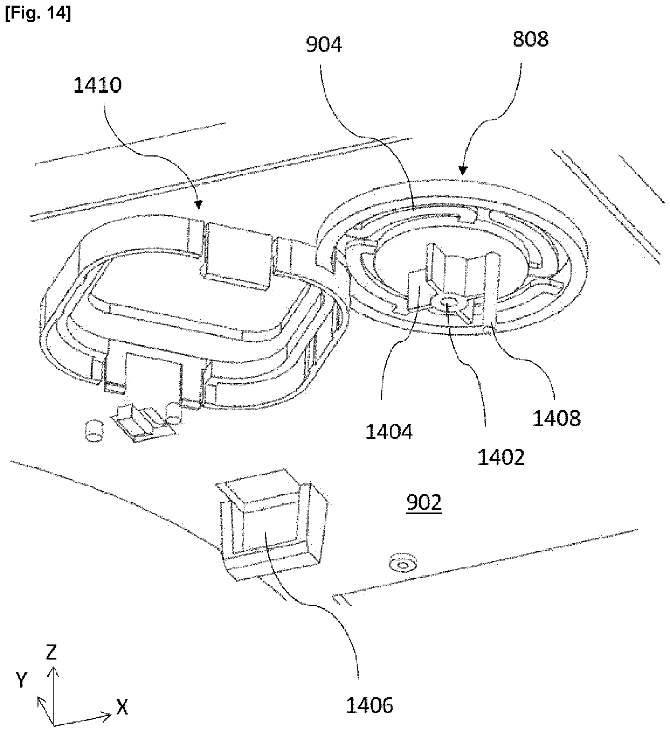

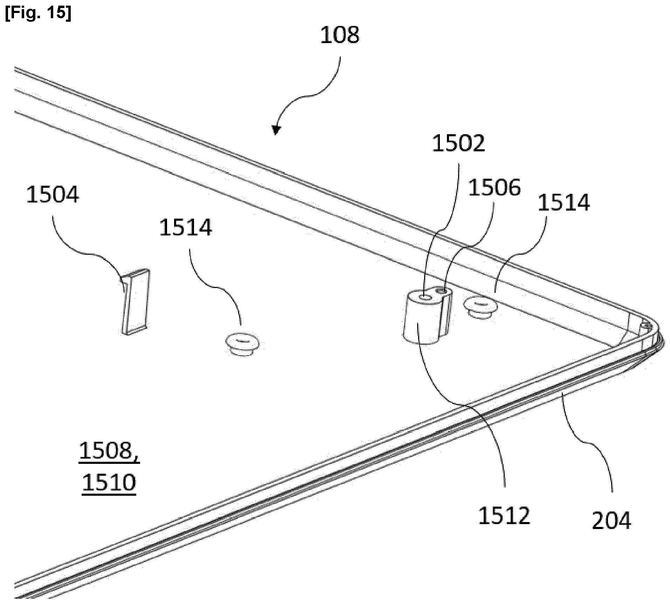

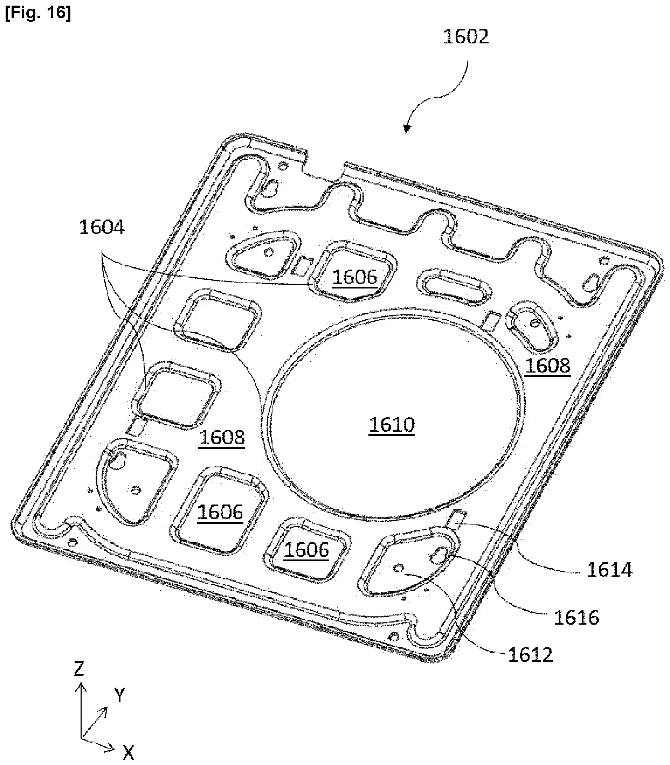

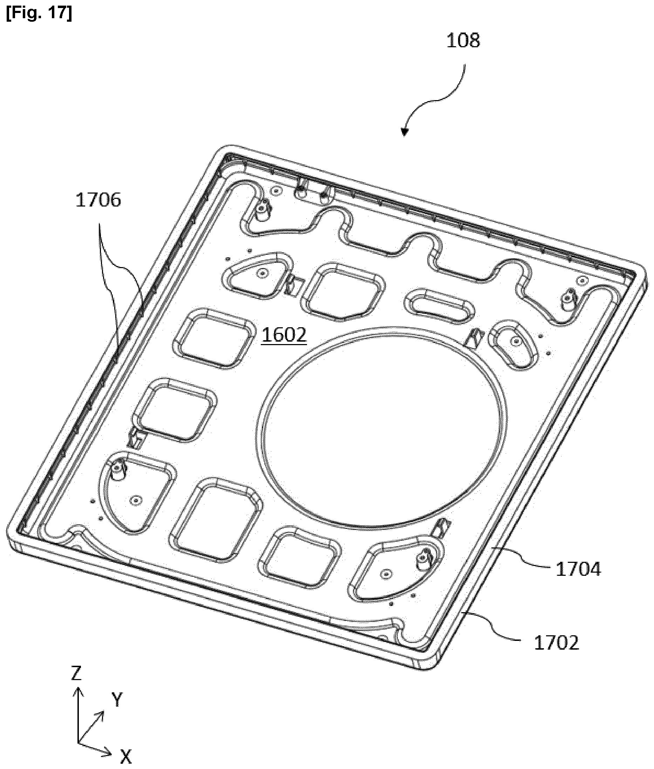

. Les figures suivantes illustrent les éléments décrits dans la présente description.
. FIG. 1 : la figure 1 représente une vue tridimensionnelle d'une station de mesure avec une poignée, selon un mode de réalisation ;
. FIG. 2 : la figure 2 représente une vue latérale de la station de la figure 1 ;
. FIG. 3 : la figure 3 représente une vue tridimensionnelle de la station de la figure 1 ; mais avec la poignée en position déployée ;
. FIG. 4 : la figure 4 représente une vue détaillée de la poignée ;
. FIG. 5 : la figure 5 représente une vue schématique de la station de mesure et de son environnement ;
. FIG. 6A : la figure 6A illustre une vue détaillée de la poignée, selon un mode de réalisation ;
. FIG 6B : la figure 6B illustre une vue de côté de la poignée sur un support de poignée de la base, selon un mode de réalisation de l'invention ;
. FIG. 7 : la figure 7 illustre une vue tridimensionnelle de la poignée, selon un mode de réalisation ;
. FIG. 8 : la figure 8 illustre une vue tridimensionnelle, en transparence, d'une plaque de support, selon un mode de réalisation ;
. FIG. 9 : la figure 9 illustre une vue de derrière d'une plaque de support, selon un mode de réalisation ;
. FIG. 10 : la figure 10 illustre un agrandissement d'une partie d'une plaque de support, selon un mode de réalisation ;
. FIG. 11 : la figure 11 illustre une vue tridimensionnelle d'une plaque de mesure, avec un agrandissement, selon un mode de réalisation ;
. FIG. 12 : la figure 12 illustre une vue de derrière d'une plaque de mesure, selon un mode de réalisation ;
. FIG. 13 : la figure 13 illustre un support de poignée avec un agrandissement, selon un mode de réalisation ;
. FIG. 14 : la figure 14 illustre un gros plan d'une plaque de support, selon un mode de réalisation ;
. FIG. 15 : la figure 15 illustre un gros plan d'un fond du socle, selon un mode de réalisation ;
. FIG. 16 : la figure 16 illustre une plaque de rigidification, selon un mode de réalisation ;
. FIG. 17 : la figure 17 illustre une plaque de rigidification avec une collerette, selon un mode de réalisation.

### Description détaillée

. Les figures 1 à 4 illustrent une représentation d'une station de mesure 100 selon au moins un mode de réalisation de la présente description. La station de mesure 100 se présente principalement sous la forme d'une base 102 sur laquelle un utilisateur peut poser ses pieds, par exemple à plat. L'utilisateur peut être sur la station de mesure ou assis sur une chaise. En position normale d'utilisation, les pieds de l'utilisateur sont posés à plat sur la station de mesure 100. L'épaisseur de la base 102 est par exemple inférieure à 10 cm, voire 6cm. La station de mesure 100 comprend un ou plusieurs capteurs 104 aptes à mesurer des informations physiologiques d'un utilisateur.

. Dans un mode de réalisation, certains capteurs 104 (par exemple des électrodes) sont montés sur un substrat 106 de la base 102, le substrat étant configuré pour recevoir les pieds d'un utilisateur. Le substrat peut être une plaque rigide, comme illustré sur les figures, et appelée plaque de mesure 106. La plaque de mesure 106 définit un plan parallèle à un plan XY. La plaque de mesure 106 peut être en verre. Néanmoins, le substrat peut être déformable sous le poids de l'utilisateur. Le substrat 106 peut être monté sur un socle 108, par exemple rigide, ou des pieds (non illustré). Dans le cas d'une base 102 fonctionnant comme un pèse-personne, des capteurs sont positionnés entre le substrat 106 et le socle 108 (architecture dite « sandwich ») ou entre le substrat 106 et les pieds (architecture dite « à pieds »). Les capteurs peuvent être des cellules de charge (généralement quatre) qui permettent d'obtenir un poids, et donc une masse d'un utilisateur. Le socle 108 peut être en métal (aluminium, acier, etc.) ou en plastique.

. Comme visible en figure 2, la station de mesure 100 comprend de plus une plaque de support 202, qui peut être solidaire de la plaque de mesure 106. La plaque de support 202 est conçue pour recevoir une partie de l'électronique de la station de mesure 100, notamment via un circuit imprimé PCB (« *printed circuit board* ») monté sur la plaque de support 202. La plaque de support 202 est donc positionnée entre le socle 108 et la plaque de mesure 106.

. Dans une architecture à pieds, on définit deux groupes en déplacement l'un par rapport à l'autre : les pieds d'une part (groupe fixe), et tout le reste d'autre part (groupe mobile). Les cellules de charges relient mécaniquement ces deux groupes. La plaque de support, si présente, est alors généralement cachée par un capot externe solidaire de la plaque de mesure. Visuellement, seule la partie mobile est généralement visible.

. Dans une architecture sandwich, on définit deux groupes en déplacement l'un par rapport à l'autre : le socle 108 (et éléments associés) d'une part (groupe fixe), et tout le reste d'autre part (groupe mobile). Les cellules de charges relient mécaniquement ces deux groupes. Visuellement, les deux groupes sont généralement visibles.

. Dans un mode de réalisation, la station de mesure 100 comprend en outre une poignée 110, apte à être saisie par une moins une main de l'utilisateur, illustrée en figures 3 et 4. La poignée 110 peut être reliée à la station de mesure par un câble 302 (visible sur la figure 3). Le câble 302 permet notamment de faire transiter des signaux électriques entre la poignée 110 et la base 102. Afin d'avoir une station de mesure 100 pratique et sans câble volant, le câble 302 peut se déployer et se rétracter (par exemple s'enrouler et se dérouler) à l'intérieur de la base 102. A cette fin, un enrouleur (visible sur la figure 8) est agencé dans un espace prévu entre le substrat 106 et le socle 108. Au moins deux positions sont ainsi définies pour la poignée : une position rangée (visible en figures 1 et 2) et une position déployée (visible en figure 3). La base 102 comprend en outre un support de poignée 112 qui peut accueillir la poignée 110 en position rangée. Le support de poignée 112 est par exemple monté sur le substrat 106. Le support de poignée 112 peut être une pièce rapportée sur la plaque de mesure 106. Par exemple, le support de poignée 112 peut être collé sur la plaque de mesure 106 (notamment lorsqu'elle est en verre) ou bien vissé. Il sera décrit plus en détail par la suite. La poignée 110 comprend elle aussi au moins un capteur 402.

. La poignée 110 est utilisée pour effectuer au moins une mesure parmi les mesures suivantes : ECG (ECG-1-voie entre les deux mains ou plusieurs voies avec d'autres membres), BIA (dites « segmentales »), IPG. Les capteurs de la poignée 110 sont choisis notamment parmi : capteur optique pour PPG et électrodes.

. La base 102 peut comprendre un affichage 114 (par exemple un écran ou un affichage à LED ou à encre électronique) pour afficher des informations à destination de l'utilisateur. L'écran 114 est affiché en pointillé sur la figure 1 car, dans l'exemple des figures, il n'est pas ou peu visible lorsqu'il est éteint.

. Le socle 108 de la base 102 peut comprendre un chanfrein 204 pour faciliter la préhension de la station de mesure 100 lorsqu'elle est sur le sol.

. Dans un mode de réalisation, la base 102 a une forme essentiellement rectangulaire dans un plan XY. La base 102 a par exemple une forme essentiellement parallélépipédique dans l'espace XYZ.

. Lorsque la station de mesure 100 est positionnée à plat, la plaque de mesure 106 est parallèle à un plan XY. La station de mesure 100 comprend une direction longitudinale dans un plan XY et une dimension transversale dans un plan XY et orthogonale à la direction longitudinale. Par hauteur, on entend la dimension selon l'axe Z (aussi appelée épaisseur) ; par largeur, il est signifié la dimension transversale selon l'axe X ; par longueur, il est signifié la dimension longitudinale selon l'axe Y. En utilisation normale, les pieds de l'utilisateur se positionnent le long de la longueur Y de la base 102. Le bord de la station de mesure 100 (ou de la base 102, ou bien encore de la plaque de mesure 106) qui est le plus proche de la partie antérieure du pied en utilisation normale (c'est à dire les doigts de pied) est appelé bord antérieur 206 et le bord opposé de la station de mesure 100 (ou de la base 102, ou bien encore de la plaque de mesure 106), qui est le plus proche de la partie postérieure du pied en utilisation normale (c'est-à-dire le talon) est appelé bord postérieur 208. On peut définir un axe médian, selon la longueur Y (longitudinale donc), autour duquel la plaque de mesure 106 est symétrique et qui permet de définir une partie gauche, destinée au pied gauche, et une partie droite, destinée au pied droit. La largeur X102 de la base 102 peut être comprise entre 330 et 400mm (par exemple environ 357mm) et la longueur de la base Y102 peut être comprise entre 300 et 360mm (par exemple environ 325mm). La longueur et/ou la largeur de la plaque de mesure 106 peuvent être légèrement moindres que celles du socle 108, de sorte que la plaque de mesure 106 soit légèrement en retrait par rapport au socle 108. Dans ce cas, la longueur et la largeur du socle 108 correspondent respectivement à la longueur et à la largeur données ci-dessus pour la base 102. Une telle conception permet de protéger la plaque de mesure 106 des chocs et des contacts avec l'environnement extérieur. La demande FR2106653 décrit une telle solution.

. Concrètement, comme illustré en figure 2 : la hauteur Z102 de la base 102 peut être comprise entre 20 et 35mm (par exemple entre 35 et 40mm) et la hauteur maximale Z100 de la station de mesure 100 peut être comprise entre 45 et 55mm (par exemple 51mm). Comme illustré sur les figures, seuls le support de poignée 112 et la poignée 110 sont en saillie selon la direction Z par rapport à la plaque de mesure 106. En détaillant les différentes dimensions : la hauteur H108 du socle 108 peut être comprise entre 10 et 20mm (par exemple 18mm), la hauteur Z202 de la plaque de support 202 peut être comprise entre 3 et 6mm (par exemple 4mm), la hauteur Z106 de la plaque de mesure 106 peut être comprise entre 4 et 8mm (par exemple 6mm).

. Le câble 302 peut avoir une longueur comprise entre 50cm et 120cm. La longueur est choisie pour que la plupart des utilisateurs puisse saisir la poignée en se tenant debout et avoir les mains vers le bas (au repos).

. La station de mesure 100 pourrait toutefois avoir des formes et/ou des dimensions différentes, pourvu que la forme et/ou les dimensions permettent l'obtention des mesures présentement décrites. En particulier, la base 102 pourrait avoir une forme ovale ou plus arrondie dans le plan XY.

. La station de mesure 100 peut avoir une masse comprise entre 3 et 6kg (par exemple entre 4 et 5kg).

. Grâce au(x) capteur(s) de la base 102 et/ou au(x) capteur(s) de la poignée 110, la station de mesure 100 peut effectuer un groupe de mesures sur l'utilisateur. En particulier, les capteurs 104 utilisés comprennent des électrodes qui sont formées à partir de chemins conducteurs d'électricité montés sur le substrat 106 et/ou la poignée 110 (inserts métalliques, dépôts métalliques, etc.). Certaines mesures peuvent ne nécessiter que des capteurs de la base 102, d'autres mesures peuvent ne nécessiter que des capteurs de la poignée 110, d'autres mesures peuvent nécessiter simultanément des capteurs de la poignée 110 et de la base 102.

. La station de mesure 100 peut ainsi effectuer un ECG à l'aide de la poignée 110 (par exemple un ECG 1 voie), ou un ECG à l'aide de la poignée 110 et de la base 102 (par exemple un ECG multi-voie, comme un ECG six-voies). La station de mesure 100 peut ainsi effectuer une analyse d'impédancemétrie corporelle BIA à l'aide de la poignée 110 et/ou de la base (BIA entre les jours et/ou BIA segmentale). La station de mesure peut ainsi effectuer un IPG dans l'arc de jambes (« *between legs* ») ou IPG dans le pied (« in the foot »).

. Les capteurs peuvent comprendre des électrodes aptes : à mesurer et/ou appliquer une tension (continue ou alternative) et/ou un potentiel (continu ou alternatif), et/ou injecter et/ou récupérer un courant (continu ou alternatif). Les fonctions de ces électrodes peuvent être choisies parmi la liste suivante : i+ et i-, pour l'injection de courant alternatif dans le corps d'un utilisateur ; V+ et V- pour mesurer une différence de potentiel dans le corps d'un utilisateur ; RA, LA et LL, pour mesurer une courant électrique traversant le corps d'un utilisateur. Les électrodes i+ et i-, V+ et V-sont utilisées pour un BIA, un IPG ou un ICG ; les électrodes RA, LL, LL sont utilisées pour un ECG.

. En particulier, la station de mesure 100 est configurée pour réaliser différentes mesures. Le nombre d'électrodes étant limité (pour des considérations de surface et de nombre), la station de mesure 100 présente un agencement particulier d'électrodes, avec un commutateur.

. Comme indiqué précédemment, les capteurs peuvent comprendre des cellules de charges, grâce auxquelles la station de mesure 100 peut mesurer un poids et effectuer un BCG.

. La poignée 110 est illustrée en détail en figure 4. La poignée 110 permet à la station de mesure 100 de réaliser une plus grande variété de mesures ou bien des mesures plus complètes, grâce à une connexion électrique avec au moins une main, voire les deux mains. En particulier, la BIA segmentale et/ou l'ECG multi-voies sont rendus possibles par l'ajout de la poignée 110 à la base 102. Les capteurs 402 de la poignée 110 comprennent par exemple des électrodes aptes à mesurer et/ou appliquer une tension et/ou un potentiel et/ou injecter et/ou récupérer un courant.

. Dans un mode de réalisation, en position rangée, la poignée 110 se trouve à proximité du bord antérieur 206. Cela signifie que le support de poignée 112 se trouve à proximité du bord antérieur 206. Par exemple, le support de poignée se trouve au plus entre 1 et 3 cm du bord antérieur 206.

. Dans un mode de réalisation, la poignée 110 comprend quatre électrodes, agencées en deux paires : une paire pour la main gauche et une paire pour la main droite. A cet effet, on nomme les électrodes de la poignée par : électrodes LH1, LH2, côte à côte sur une partie gauche de la poignée 110 et électrodes RH1, RH2, côte à côte sur une partie droite de la poignée (par partie gauche, respectivement droite, il est entendu la partie de la poignée destinée à être au contact de la main gauche, respectivement droite). « Côte à côte » signifie ici avec un espace entre les électrodes, pour isoler les électrodes les unes des autres. Les électrodes sont donc disposées à la suite les unes des autres entre deux extrémités de la poignée 110. Lorsque la poignée 110 est rectiligne, les électrodes sont disposées à la suite le long de la direction principale de la poignée 110. Les électrodes LH1 et RH1 sont positionnées axialement du côté d'une extrémité de la poignée ; les électrodes LH2, RH2 sont positionnées axialement du côté du centre de la poignée. On a donc, dans l'ordre, les électrodes suivantes : LH1, LH2, RH2, RH1.

. Dans un mode de réalisation, les capteurs 402 de la poignée 110, lorsqu'ils sont des électrodes, sont réalisés sous la forme de plusieurs inserts métalliques dans la poignée 110. Parmi les matériaux utilisables pour les inserts métalliques, on peut citer l'acier inoxydable, le titane, le laiton, l'ITO ou des plastiques conducteurs. Pour le traitement et/ou l'acquisition de signaux, en particulier de l'ECG, la poignée 110 peut comprendre de l'électronique de traitement (amplificateur opérationnel suiveur, etc.), notamment pour l'ECG. Il est généralement préférable d'amplifier le signal au plus près des électrodes car le câble peut capter du bruit ambiant.

. La figure 5 illustre une vue schématique de l'architecture globale 500 dans laquelle la station de mesure 100 peut être insérée. Cette architecture globale forme un système comprenant la station de mesure 100. En particulier, la station de mesure 100 peut communiquer avec des dispositifs tiers via un réseau de communication 510, qui est par exemple un réseau sans-fil (notamment un réseau compatible avec au moins l'un des protocoles de communication suivants : BlueTooth, Wi-Fi, cellulaire, etc.). Les dispositifs tiers peuvent comprendre un serveur 520 et un terminal mobile 530 (smartphone, etc.). Le serveur 520 peut comprendre une circuiterie de contrôle 522, incluant un processeur 524 et une mémoire 526, et une interface entrée/sortie (« input/output », I/O) 528, qui permet à la circuiterie de contrôle de recevoir et d'envoyer des données vers le réseau de communication 510. Le terminal mobile 530 peut comprendre une circuiterie de contrôle 532, incluant un processeur 234 et une mémoire 236, un comprendre une interface entrée/sortie (« input/output », I/O) 538, qui permet à la circuiterie de contrôle de recevoir et d'envoyer des données. Le serveur 520 est un serveur distant, par exemple situé dans un centre de données (data center). Le terminal mobile 530 comprend en outre une interface d'utilisateur 540 (« user interface », UI) configurée pour afficher des informations à l'utilisateur et lui permettre, le cas échant, d'entrer des informations (comme la taille, le sexe, etc.). En particulier, la circuiterie de contrôle 532 est configurée pour faire fonctionner une application gérant l'environnement de la station de mesure 100. Le terminal mobile 530 est un objet personnel de l'utilisateur, généralement à proximité de ce dernier.

. La station de mesure 100 peut communiquer avec le serveur 520 et/ou le terminal mobile 530. Dans un mode de réalisation, la station de mesure 100 peut communiquer directement avec le terminal mobile 530, par exemple via Bluetooth ou Bluetooth Low Emission (BLE). Cette communication peut être mise à en oeuvre à l'installation de l'appareil de mesure 100, notamment pour l'appairer avec le terminal mobile 530 et/ou pour configure une connexion au serveur 520 qui ne transite pas par le terminal mobile 530 et/ou en en secours d'une communication avec le serveur 520 défaillante. Dans un mode de réalisation, la station de mesure 100 peut communiquer directement avec le serveur 520, sans transiter par le terminal mobile 530. Cette communication permet à l'utilisateur d'utiliser la station de mesure même sans avoir son terminal mobile 530 à proximité.

. La station de mesure 100 comprend elle aussi une circuiterie de contrôle 550 avec un processeur 552 et une mémoire 554, et une interface entrée/sortie 556 (« input/output », I/O), qui permet notamment à la circuiterie de contrôle de recevoir et d'envoyer des données au réseau de communication 510. Le processeur 552 est configuré pour notamment traiter des données obtenues par les capteurs 104. En particulier, le processeur 552 peut exécuter des instructions d'un programme stocké dans la mémoire 554. La circuiterie de contrôle 550 peut comprendre un microcontrôleur, qui intègre le processeur 552, la mémoire 554 et l'interface entrée/sortie 556. La circuiterie de contrôle 550 peut en outre comprendre un dispositif frontal analogique (« Analog Front End », AFE). La circuiterie de contrôle 550 peut aussi comprendre un convertisseur analogique/digital (« Analog to Digital Converters », ADC). La station de mesure 100 comprend une source de tension 558 (par exemple continue) et une source de courant 560 (par exemple alternatif). La station de mesure 100 comprend aussi un voltmètre 562 (ou tout système permet de mesurer une tension). Le voltmètre 562 peut être intégré à l'AFE. La source de courant 560 peut être intégré à l'AFE et la source de tension 558 peut être intégrée au microcontrôleur MCU (par exemple via un convertisseur digital/analogique, « digital to analog converter » DAC). Certains capteurs 104 (en particulier les capteurs 402 de la poignée 110 de la figure 4 ou les électrodes 602 de la poignée 110 sur la figure 6A) sont reliés à la circuiterie de contrôle 550 (par exemple au MCU ou à l'AFE). La station de mesure 100 comprend une batterie 564, apte à alimenter en énergie les différents composants de la station de mesure 100.

. La circuiterie de contrôle 550 et d'autres composants électroniques peuvent être montés sur un circuit imprimé PCB (« *printed circuit board* »), qui est par exemple attaché à la plaque de support 202. Des connecteurs relient les chemins conducteurs d'électricité de la plaque de mesure au PCB. Afin de pouvoir modifier les connexions des électrodes aux différents composants de la station de mesure 100, la station de mesure comprend un commutateur 566. Le commutateur 566, qui peut comprendre une pluralité d'interrupteurs pilotés par le MCU, sera décrit plus en détail par la suite.

. La circuiterie de contrôle 550 comprend par exemple un système d'acquisition d'ECG, un système d'impédancemétrie (pour BIA ou IPG), un système d'ESC (pour l'ESC). Le système d'acquisition d'ECG comprend des électrodes (représentées par 602 sur les figures 6a et 6b et 402 sur la figure 4) et un circuit électrique ECG 568 (qui intègre notamment différents étages d'amplification et/ou de filtrage et un démodulateur) ; le système d'impédancemétrie comprend notamment des électrodes (représentées par 602 sur les figures 6a et 6b et 402 sur la figure 4), la source de courant 560, le voltmètre 562 et un circuit électrique d'impédancemétrie 570 qui relie les électrodes à la source de courant et au voltmètre (qui intègre différents étages d'amplification et/ou de filtrage) : le système d'ESC comprend des électrodes, la source de tension 558 et un circuit électrique d'ESC 572 (qui intègre différents composants électroniques, dont des résistances). Le commutateur 566 permet de relier les électrodes notamment aux différents circuits 568, 570, 572 précités, ou bien de déconnecter toutes les électrodes de la circuiterie de contrôle 550.

. La circuiterie de contrôle 550 se trouve essentiellement dans la base 102, à l'exception de notamment quelques composants (amplification, filtrage et interrupteurs) disposés dans un PCB dans la poignée 110, pour traiter les signaux avant de les faire transiter par le câble 302.

. Comme indiqué auparavant, la station de mesure 100 comprend aussi l'affichage 114, telle qu'un écran (OLED/PMOLD, Retina, etc.), pour afficher à l'utilisateur des informations. Alternativement, la station de mesure 100 ne comprend pas d'affichage.

. La poignée 110 est illustrée en détail en figures 4, 6 et 7. La poignée 110 permet à la station de mesure 100 de réaliser une plus grande variété de mesures ou bien des mesures plus complètes, grâce à une connexion électrique avec au moins une main, par exemple les deux mains. En particulier, la BIA segmentale et/ou l'ECG multi-voies sont rendus possibles par l'ajout de la poignée 110 à la base 102. Les capteurs 402 de la poignée 110 comprennent par exemple des électrodes 602 aptes à mesurer et/ou appliquer une tension et/ou un potentiel et/ou injecter et/ou récupérer un courant.

. Comme décrit auparavant, dans un mode de réalisation, la poignée 110 comprend quatre électrodes, agencées en deux paires : une paire pour la main gauche et une paire pour la main droite. A cet effet, on nomme les électrodes de la poignée par : électrodes LH1, LH2, côte à côte sur une partie gauche de la poignée 110 et électrodes RH1, RH2, côte à côte sur une partie droite de la poignée (par partie gauche, respectivement droite, il est entendu la partie de la poignée destinée à être au contact de la main gauche, respectivement droite ; et par côte à côte il est inclus un espacement entre les électrodes). Les électrodes LH1 et RH1 sont positionnées axialement du côté d'une extrémité de la poignée ; les électrodes LH2, RH2 sont positionnées axialement du côté du centre. On a donc, dans l'ordre, les électrodes suivantes : LH1, LH2, RH2, RH1. Le câble 302 entre dans la poignée entre les électrodes LH1, LH2 d'un côté et les électrodes RH1, RH2 de l'autre.

. Comme illustré en figure 6B, les électrodes 602 sont positionnées en décalé par rapport à l'endroit où le câble 302 rentre dans la poignée 110. Dans le cas d'une poignée de forme cylindrique d'axe A, parallèle à la direction transversale X de la station de mesure 100 en position rangée, où la position d'insertion du câble 203 dans la poignée 110 définit 0°, les électrodes s'étendent sur une surface externe de la poignée 110 comprise entre -45° et +90° (dans le sens trigonométrique par rapport au repère orthonormé XYZ), voire comprise entre -30° et 80°. Dans le mode de réalisation des figures, les électrodes s'étendent de -30° à +79° (angle couvert par les électrodes de 109°, à plus ou moins 1°). Les angles sont définis dans une section transversale de la poignée 110 (un plan de coupe orthogonal à l'axe A donc un plan de coupe en YZ lorsque la poignée 110 est positionnée dans le support de poignée 112). Les angles positifs entre 0 et +180° sont orientés vers le bord le plus proche de la base 102. En d'autres termes, en position rangée, les électrodes s'étendent sur la poignée davantage du côté du bord antérieur 206 de la base 102 que du côté du bord postérieur 208.

. Plus généralement, pour toute forme de la poignée (rectiligne, légèrement courbée, non-cylindrique, etc.), on peut définir le positionnement des électrodes comme suit. La poignée 110 comprend une ligne médiane 610, qui est une ligne passant par le centre du volume définit par la poignée 110, c'est-à-dire une ligne définie par les isobarycentres des sections transversales successives de la poignée 110 (isobarycentre de la courbe définie par la surface externe de la poignée 110 dans une section transversale). La poignée 110 comprend une ligne inférieure 612, qui est une ligne définie, pour des sections successives de la poignée 110, par l'ensemble des points de la surface externe de la poignée 110 qui sont le plus proches de la plaque de mesure 106 (en position rangée). Pour chaque section transversale à la ligne médiane, on définit l'angle passant par la ligne médiane et une extrémité des électrodes 602 sur une surface externe de la poignée 110. L'angle 0° est défini comme celui entre la médiane et la ligne inférieure ; les angles positifs sont définis à partir de 0° en allant du côté du bord antérieur 206 et les angles négatifs sont définis à partir de 0° en allant en direction du bord postérieur 208 (les angles s'étendent donc de -180° à +180°). Le câble 302 s'insère dans la poignée 110 à l'angle 0° (le point d'entrée du câble 302 dans la poigné 110 est donc à 0°). Pour chaque section transversale à la ligne médiane 610 qui inclut une portion d'électrode 602, l'électrode 602 s'étend au plus sur une surface de la poignée comprise entre -45° et +90°, notamment entre -30° et 80°. Dans un mode de réalisation, les électrodes s'étendent de -30° à +79° (angle total couvert par les électrodes de 109°, à plus ou moins 1°).

. Ce positionnement des électrodes présente plusieurs avantages. Lors de la préhension de la poignée 110, le câble 302 reste en général vers le bas, aligné avec la verticale Z. Par conséquent, premièrement, avec une préhension naturelle et intuitive, le positionnement des électrodes permet un contact optimisé avec les doigts, en permettant un contact des électrodes avec tous les doigts : les phalangines des doigts les plus longs, les phalangines ou les phalangettes des doigts les plus courts. Deuxièmement, en évitant de venir mettre en contact les phalanges des doigts, la poignée 110 peut être utilisée avec des bagues. Troisièmement, les électrodes sont positionnées vers le bas, c'est-à-dire qu'elles orientées vers le sol : le poids de la poignée, du câble et éventuellement une force de rapport du câble participent à maintenir les électrodes en contact avec les doigts qui s'enroulent sous la poignée 110 et donc sur les électrodes. Quatrièmement, la position des quatre électrodes le long de la direction A permet aux électrodes d'être toutes en contact avec une main dans le cadre d'une préhension naturelle et intuitive de la poignée 110.

. La poignée 110 peut comprendre un corps principal 404 sur lequel sont montés les électrodes et les autres composants (électronique, attache, etc.). Le corps principal 404 peut avoir une forme allongée selon un axe longitudinal (qui est parallèle le long de la direction X en position rangée), par exemple généralement cylindrique et plus précisément cylindrique de révolution. La forme est choisie pour être préhensible par un utilisateur. Pour faciliter l'assemblage, le corps principal 404 peut comprendre une première partie 702 et une deuxième partie 704. La première partie 702 peut recevoir les électrodes 402 et la deuxième partie 704 peut être montée sur la première partie 702. En particulier, la deuxième partie 704 peut comprendre une section cylindrique (par exemple de révolution) avec deux extrémités (par exemple plates ou arrondies). Une zone de la paroi latérale de la section cylindrique peut être absente (par exemple d'une extrémité à l'autre) afin de pouvoir loger la première partie 702 du corps principal 404. Une matière plastique peut être utilisé pour le corps principal, afin de garder la poignée légère (en cas de chute) et de bien isoler les électrodes 402 les unes des autres.

. Dans un mode de réalisation, les électrodes 402 sont réalisées sous la forme de plusieurs inserts métalliques dans le corps principal 404. Parmi les matériaux utilisables pour les inserts métalliques, on peut citer l'acier inoxydable, le titane, le laiton, l'ITO (oxyde d'indium-étain), nickel (ou alliage de nickel) ou des plastiques conducteurs. Pour le traitement et/ou l'acquisition de signaux, en particulier de l'ECG, la poignée 110 peut comprendre de l'électronique de traitement (amplification, filtrage, etc.), notamment pour l'ECG. Il est généralement préférable d'amplifier le signal au plus près des électrodes car le câble peut capter du bruit ambiant.

. En position rangée, la première partie 702 est en regard de la base 102 et la deuxième partie 704 est visible. Les électrodes 402 sont ainsi disposées contre le support de poignée 112.

. Dans un mode de réalisation, la poignée 110 est démontable, notamment à des fins de réparation. A cette fin, la poignée 110 comprend un système de fixation 604 pour relier ladite poignée 110 au câble 302. Le système de fixation 604 permet de simplement changer la poignée 110 sans changer le câble 302. Le système de fixation 604 peut être monté dans la première partie 702 de la poignée. Cela positionne les électrodes du côté du câble 302, ce qui assure une position naturelle de contact entre les électrodes 402 et les doigts de l'utilisateur. Le système de fixation 604 peut comprendre une mâchoire 606, 608 qui vient s'arranger autour d'un stoppeur monté sur le câble 302.

. Dans un mode de réalisation, le support de poignée 112 et la poignée 110 comprennent un système d'attache magnétique (par exemple au moyen d'au moins un aimant permanent). Un matériau magnétique est prévu dans le support 112 et un aimant est monté dans la poignée 110 (ou inversement). Les systèmes d'attache par aimant servent aussi de détrompeur lors du positionnement de la poignée 110 sur le support de poignée 112 (la partie gauche de la poignée ne peut pas être posée sur la partie droite du support de poignée à cause des polarités). Des aimants en néodyme peuvent être utilisés.

. Comme indiqué auparavant, le câble 302 peut se ranger dans la base 102 (en position rangée, visible en figures 1 et 2) et être hors de la base 102 (en position déployée, visible en figure 3).

. Afin que le câble 302 se mette plus naturellement en position rangée, l'extrémité du câble 302 fixé à la poignée 110 s'étend orthogonalement par rapport à une surface de la poignée. En particulier, la poignée illustrée sur les figures a une forme cylindrique : le câble 302 s'étend donc radialement par rapport au cylindre.

. Le changement de position entre une position rangée et une position déployée implique donc un déplacement du câble 302 par rapport à la base. Il est souhaitable, pour des raisons de sécurité et de praticité, que le câble 302 soit quasiment intégralement logé dans la base 102 en position rangée. Pour que cela soit possible sans effort particulier de l'utilisateur, un mécanisme de rappel 802 est monté dans la balance. Dans un mode de réalisation, ce mécanisme de rappel est un enrouleur, c'est-à-dire que le câble 302 s'enroule et se déroule lorsque l'utilisateur se prépare à utiliser la poignée 110. La figure 8 illustre le positionnement d'un tel enrouleur 802 (illustré sans son capot) dans la base 102. L'enrouleur 802 est par exemple monté sur la plaque de support 202 qui peut comprendre une ouverture traversante 902 pour loger l'enrouleur (pour minimiser l'épaisseur de la base 102). L'enrouleur 802 peut comprendre des pattes de fixation qui sont vissées à la plaque de support 202. L'enrouleur 802 comprend par exemple une portion fixe et une portion mobile, avec un ressort entre les deux pour avoir une fonction de rappel lorsque la poignée 110 est en position déployée ou dans une position intermédiaire. Le document WO2021/082876 décrit un exemple de mécanisme d'enrouleur intégré dans un pèse-personne.

. Pour guider le câble 302 entre l'enrouleur 802 et la poignée 110, différentes solutions sont proposées, qui peuvent être mises en oeuvre ensemble ou séparément.

. La plaque de mesure 106 comprend un orifice traversant 1102 pour le passage du câble 302. L'orientation radiale du câble 302 par rapport au cylindre de la poignée permet au câble de s'insérer directement dans l'orifice traversant 1102 de la plaque de mesure 106. L'orifice traversant 1102 est par exemple cylindrique (donc tout droit). L'orifice traversant 1102 peut être orthogonal à plaque de mesure 106 (parallèle à l'axe Z donc, et pas en biais), pour faciliter le tirage de la poignée 110, par exemple un exemple de forme cylindrique droite. L'orifice traversant 1102 peut avoir une section équivalente à celle du câble 302 (avec un léger jeu fonctionnel). Par exemple l'orifice traversant 1102 est de forme cylindrique. Par exemple, la dimension maximale en section du trou est de 2 cm ou 1 cm.

. En particulier, la plaque de support 202 et la plaque de mesure 106 comprennent chacune un orifice traversant 804 et 1102, en regard l'un de l'autre, pour le passage du câble 302.

. L'enrouleur 802 enroule et déroule le câble 302 dans le plan XY (essentiellement selon Y) alors que la poignée 110 (et donc la partie du câble située hors de la base 102) se déplace selon la direction Z. Par conséquent, le câble 302 doit effectuer un ou plusieurs changements de direction. A proximité de l'orifice 804 de la plaque de support 202, un rouleau 1002 (ou une poulie) est agencé pour faciliter le changement de direction du câble 302 (et éviter les frottements qui à la fois détériorent le câble 302, la plaque de support 202 et/ou le support de poignée 112 et gênent l'enroulement et le déroulement du câble. Le rouleau 1002 peut être un rouleau rotatif ou simplement un rouleau avec un revêtement lisse. Comme illustré sur la figure 10 (qui illustre une portion de la plaque de support 202), le rouleau 1002 peut être monté sur la plaque de support 202 (sur la face inférieure 1004, c'est-à-dire la face qui est en direction du sol lorsque la station de mesure 100 est en position d'utilisation). Le rouleau 1002 est agencé pour être, selon la direction Z, entre le câble 302 et la plaque de support 202 (ou la plaque de mesure 106). Un deuxième rouleau (non représenté) peut être prévu entre le rouleau 1002 et l'enrouleur 802 pour canaliser le câble. Le deuxième rouleau peut être agencé de sorte que le câble 302 soit entre ledit deuxième rouleau et la plaque de mesure. Alternativement, le deuxième rouleau est en regard du premier rouleau 1002. Les deux rouleaux sont donc de part et d'autre du câble 302 (le long de la direction Z). A la place du rouleau, une poulie ou tout élément remplissant une fonction similaire peut être utilisé.

. Pour limiter les frottements au passage de l'orifice 804 de la plaque de support 202 et pour adoucir l'angle fait par le câble 302, les bords de l'orifice sont chanfreinés (chanfrein 1006 visible en figure 10), au moins du côté de la face inférieure 1004. De la même façon, pour limiter les frottements au passage de l'orifice 1102 de la plaque de mesure 106, les bords de l'orifice 1102 sont chanfreinés (voir figure 11 : chanfrein 1106 visible sur la face supérieure 1104 de la plaque de mesure 106, c'est-à-dire la face visible, sur laquelle l'utilisateur pose ses pieds). Comme la plaque de mesure 106 peut être en verre et donc coupante, l'orifice 1102 est chanfreiné des deux côtés (voir figure 12, qui présente un grossissement en perspective : chanfrein 1202 visible sur la face inférieure 1204 de la plaque de mesure 106, c'est-à-dire la face invisible, qui est en regard de la plaque de support 202).

. Dans l'exemple illustré sur les figures, le support de poignée 112 est une pièce posée, par exemple par collage, sur la face supérieure 1104 de la plaque de mesure. Le support de poignée 112 présente ici une forme allongée. Dans le cadre de l'invention, le support de poignée 112 forme un berceau 1302, de type berceau arrondi. Le berceau peut avoir une forme complémentaire à une portion de la poignée 110 (ici la première partie 702 de la poignée 110. Dans l'exemple illustré en figure 6B, le berceau 1302 est arrondi autour d'un axe de direction X. La poignée 110, lorsque positionnée sur le support de poignée 112 ne touche pas la plaque de mesure 106.

. Afin notamment que le câble 302 soit entièrement invisible en position repliée, le support de poignée 112 comprend un orifice traversant 1304, en regard avec l'orifice 1102 de la plaque de mesure 106. L'orientation radiale du câble 302 par rapport au cylindre de la poignée permet au câble de s'insérer directement dans l'orifice traversant 1304 du support de poignée 112. L'orifice traversant 1304 est par exemple cylindrique (donc tout droit). L'orifice traversant 1304 peut être orthogonal à plaque de mesure 106 (parallèle à l'axe Z donc, et pas en biais), pour faciliter le tirage de la poignée 110, par exemple un exemple de forme cylindrique droite. L'orifice traversant 1304 peut avoir une section équivalente à celle du câble 302 (avec un léger jeu fonctionnel). Par exemple l'orifice traversant 1304 est de forme cylindrique. Par exemple, la dimension maximale en section du trou est de 2 cm ou 1 cm.

. L'orifice traversant 1304 est avantageusement positionné au fond du berceau 1302. Le fond du berceau est défini comme la position la plus basse selon la direction Z, c'est-à-dire la position la plus proche de la plaque de mesure 106. De plus, l'orifice traversant 1304 peut être centré dans le berceau selon l'axe Y. Le déplacement du câble 302 peut, à partir de l'orifice traversant 1304 du support de poignée 112, devenir un peu plus erratique du fait de l'utilisation de la poignée 110 par un utilisateur. Toujours pour limiter les frottements, l'orifice traversant 1304 du support de poignée 112 est chanfreiné. En particulier, les bords de l'orifice traversant 1304 sont chanfreinés dans la direction Y et, peuvent être chanfreinés selon la direction X (même chanfreiné sur tout le tour). Un chanfrein 1306 est ainsi prévu sur au moins un des deux bords opposés selon Y de l'orifice traversant 1304. De la même façon, un chanfrein 1306 peut ainsi être prévu sur au moins un des deux bords opposés selon Y de l'orifice traversant 1304.

. Le berceau 1302 peut s'étendre sur un angle au moins égal à la valeur négative de la position des électrodes 602 sur la poignée 110 par rapport au fond du berceau (qui correspond à la position angulaire de l'orifice traversant 1304 et donc de l'emplacement du câble 302 sur les figures, c'est-à-dire -30° dans l'exemple illustré. En d'autres termes, dans l'exemple donné, dans une section transversale (un plan de coupe en YZ), le berceau s'étend sur 30° entre son extrémité et le fond du berceau (qui correspond ici à position angulaire de l'orifice traversant 1304. En ayant un berceau qui s'étend sur une portion angulaire (autour d'un axe parallèle à la direction X) au moins égale à celle des électrodes, ces dernières sont invisibles lorsque la station de mesure 100 est observée depuis le bord postérieur 208, ce qui correspond à son positionnement le plus fréquent chez l'utilisateur puisque le bord antérieur 206 est généralement contre un mur) car elles sont cachées par le berceau 1302 (voir figure 6B). Cette dissimulation permet d'augmenter la rétention de la station de mesure 100 (c'est-à-dire le fait que la plupart des utilisateurs de la station de mesure 100 continue à l'utilisateur après une période donnée) car l'utilisateur n'a pas un sentiment d'utiliser un produit ayant un aspect médical ou paramédical.

. Dans le cadre de l'invention, le berceau s'étend sur moins de 90°, c'est-à-dire sur un angle au plus égal à 90°, voire au plus égal à 60°, voire 45°, de part et d'autre d'un fond du berceau (qui est défini comme le 0° - l'orifice traversant se trouvant lui aussi à 0°), afin de ne pas gêner la préhension de la poignée. Les doigts peuvent alors se glisser sous la poignée pour la sortir du support de poignée 112. La valeur de 45° permet une préhension facilitée tout en dissimulant les électrodes.

. Grâce au support de poignée 112, la conception et la fabrication de la plaque de mesure 106 sont simplifiées. A l'exception de l'orifice traversant 1102, la plaque de mesure 106 peut être similaire à celle d'une station de mesure sans poignée (d'où des simplifications des procédés pour avoir plusieurs modèles différents, avec et sans poignée, par exemple). De plus, en disposant la poignée 110 sur un support de poignée 112 disposé sur la plaque de mesure 106, la poignée 110 est facilement préhensible par un utilisateur, notamment en surélevant la position rangée par rapport à la plaque de mesure 106 le long de la direction Z (c'est-à-dire que l'intégralité de la poignée est surélevée par rapport à la plaque de mesure 106). La forme en berceau 1302 permet à la poignée 110 de se ranger facilement et ainsi de pas être posée par terre où elle risque de s'abîmer, d'abîmer la base 102 ou le sol sur lequel elle est posée. La disposition du câble 310 selon la direction verticale 2, en alignement avec les orifices traversants 1304 et 1102 du support de poignée 112 et de la plaque de mesure 106 contribue à simplifier la mise en position rangée de la station de mesure 100 car il suffit d'accompagner la force de rappel de l'enrouleur 802 pour que la poignée 110 se positionne dans le berceau 1302 (notamment du fait du positionnement de l'orifice traversant 1304 au fond du berceau 1302).

. Dans la description ci-dessus et sur les figures, le support de poignée est continu le long de la direction X. Toutefois, il peut être discontinu le long de cette direction. Par exemple, la forme en berceau peut être générée par deux ou trois soutiens, aux extrémités et au milieu. Cette configuration dissimule moins le câble et les électrodes de la poignée toutefois.

. Alternativement, la station de mesure 100 ne comprend pas de support de poignée et la poignée est posée directement sur la plaque de mesure 106. Cette dernière peut être usinée ou moulée pour avoir une forme permettant de recevoir et maintenir la poignée 110 en place. Par exemple, une forme en berceau peut être fait dans le verre. L'orifice traversant 1102 est alors au fond du berceau. Le système d'attache magnétique peut alors être prévu dans la poignée et sous la plaque de mesure.

. Dans un mode de réalisation, la station de mesure 100 a une fonction balance, afin de mesurer un poids et donc une masse d'un utilisateur. Les capteurs de la base 100 comprennent alors au moins un capteur de poids ou un capteur de masse. En particulier, une solution connue consiste à utiliser des cellules de charges, qui convertissent une déformation d'un élément en un signal électrique. Lorsque l'utilisateur monte sur la plaque de mesure 106, sa masse génère un effort qui est repris par la plaque de mesure 106, puis transmis (directement par contact ou indirectement via une pièce intermédiaire comme la plaque de support 202) à au moins une cellule de charge, puis ensuite le contact est repris par le socle 108 (ou les pieds pour l'architecture à pied). Quatre cellules de charges peuvent être prévues, à proximité de chaque angle de la base 102. Lorsque la base 102 n'est pas rectangulaire, les cellules de charge peuvent être disposées régulièrement par rapport à la géométrie (par exemple avec une symétrie autour d'axes médians).

. Dans un mode de réalisation illustré en figures 8 et 9, la plaque de support 202 comprend des orifices 812 aptes à loger (sans contact) les cellules de charge. De la sorte, la plaque de support 202 n'interfère pas dans la reprise d'effort entre la plaque de mesure 106 et les cellules de charge.

. Toutefois, dans l'architecture sandwich, le déplacement dans le plan XY de l'ensemble mobile (plaque de mesure 106, plaque de support 202 et d'autres composants, notamment électroniques) doit être limité par rapport à l'ensemble fixe (le socle 108 essentiellement). La limitation de ce déplacement ne doit néanmoins pas contaminer la mesure de poids, c'est-à-dire qu'elle ne doit pas interagir avec le déplacement selon la direction en Z ni reprendre d'effort selon la direction Z. Le document PCT/EP2017/050178 décrit une solution satisfaisante à ce problème. La présente description propose un autre mode de réalisation. La plaque de support 202 comprend au moins un îlot 808, par exemple un îlot 808 par cellule de charge (quatre sur les figures, un à proximité de chaque cellule de charge). L'ilot 808 est une pièce de la plaque de support 202 qui est relié à un corps principal 902 de la base de plaque support 202 par un membre élastique 904. Le membre élastique 904 a pour fonction de limiter le déplacement de l'ensemble mobile en XY en gênant le plus moins possible le déplacement en Z. Le membre élastique 904 peut comprendre une ou plusieurs pattes s'étendant entre l'îlot 808 et le corps principal 902 de la plaque de support 202. Sur les figures, chaque membre élastique 904 comprend quatre pattes identiques. Chaque îlot 808 est logé dans un orifice traversant formé dans le corps principal 902. L'îlot 808 peut avoir une forme circulaire, carrée, rectangulaire ou autre (polygonal par exemple ou de révolution) et l'orifice traversant une forme similaire mais homothétiquement plus large.

. Chaque îlot 808 comprend un orifice d'attache 1402, par exemple sous forme d'un trou taraudé, qui est apte à recevoir une tige, par exemple une vis, qui vient solidariser le socle 108 avec l'ilot 808 (via un orifice d'attache 1502 dans le socle 108). La vis peut être taraudeuse. L'orifice d'attache 1402 peut être prévu dans une saillie 1404, qui permet à la fois de limiter la longueur de la tige et d'augmenter localement l'épaisseur de l'îlot 808 (plus de rigidité dans la liaison avec la tige).

. Néanmoins, si un utilisateur saisit la station de mesure 100 par la plaque de mesure 106 (qui fait partie de l'ensemble mobile), tout le poids de la partie fixe va passer par la tige et l'îlot 808. Le ou les membres élastiques 904 ne sont toutefois pas assez solides pour reprendre cette effort (on rappelle qu'ils ne sont pas censés interférer dans la mesure du poids). La plaque de support 202 et le socle 108 comprennent alors une butée de déplacement maximal en Z. Comme illustrée sur les figures 14 et 15, la butée peut prendre la forme d'une patte 1504 du socle 108, en forme de L (avec une portion en Z est une portion dans un plan parallèle au plan XY), qui coopère avec un réceptacle 1406 de la plaque de support 202 qui comprend une portion dans un plan parallèle au plan XY de sorte qu'il y a butée quand le déplacement en Z est trop important. Par exemple, le déplacement en Z autorisé est inférieur à 1mm.

. L'assemblage de la plaque de support 202 sur le socle 108 peut se faire par positionnement de plaque support 202 (ou plus généralement de l'ensemble mobile) au-dessus du socle. Il faut alors que les orifices d'attache 1502 du socle et les orifices d'attache 1402 soient tous alignés deux à deux pour pouvoir insérer la tige (par exemple pour insérer une vis). Cet alignement peut faire perdre quelques secondes à un opérateur. Afin de simplifier le processus, la station de mesure 100 peut comprendre un guide entre l'ensemble mobile et l'ensemble fixe. Le guide peut prendre la forme d'une tige de guidage 1408 s'étendant en Z et apte à coopérer avec un orifice de guidage 1506 du socle 108. La tige de guidage 1408 peut être située à proximité (à une distance D) de l'orifice d'attache 1402 de l'îlot 808 correspondant (par exemple moins de 1 cm) afin que l'influence des jeux de fabrication soit minime. De la même façon, l'orifice 1502 du socle 108 est à proximité de l'orifice de guidage 1506 (la même distance D). La tige de guidage 1408 vient en saillie en Z par rapport au niveau de l'orifice d'attache 1402. En particulier, même si l'îlot 808 est configuré pour être solidaire en déplacement en XY du corps principal 902, les membres élastiques 904 confèrent de fait une élasticité en XY ; pour contrecarrer ce déplacement indésirable qui peut perturber l'assemblage, la tige de guidage 1408 est monté sur ledit îlot 808. La tige de guidage 1406 permet aussi de bloquer la rotation de l'îlot 808 pendant le vissage.

. Inversement, le socle 108 pourrait présenter la tige de guidage et l'îlot 808 l'orifice de guidage.

. Un orifice 1410 est prévu à proximité de l'îlot 808 pour loger la cellule de charge, afin qu'elle soit positionnée entre la plaque de mesure 106 et le socle 108.

. Les figures 15 à 17 illustrent le socle 108 ou certaines pièces formant le socle 108. Dans un mode de réalisation, le socle est mono-pièce (par exemple en métal pour être suffisamment rigide). Néanmoins, un tel socle est lourd et coûteux. Pour pallier ces difficultés, les différentes fonctions du socle (maintien structurel de la station de mesure 100, fonction esthétique, fonction d'étanchéité, fonction de solidarisation des pièces, fonction de pesage, etc.), le socle 108 peut être constitué de plusieurs pièces. Dans le mode de réalisation illustré sur les figures, le socle 108 comprend au moins un cadre 1508 et une plaque de rigidification 1602.

. La plaque de rigidification 1602, qui s'étend dans un plan XY, est disposée entre le cadre 1508 (ou à l'intérieur d'un volume ouvert défini par ce dernier) et la plaque de support 202. Les cellules de charge reposent directement sur la plaque de rigidification 1602 qui assure alors la rigidité nécessaire pour avoir une mesure de poids correcte, même sur un sol mou (moquette par exemple) ou non plan (parquet ancien par exemple). La plaque de rigidification 1602 peut être en métal, par exemple acier ou aluminium. L'aluminium présente l'avantage d'être léger. Comme illustré sur la figure 16, la plaque de rigidification 1602 présente des nervures 1604 permettant, à quantité égale de matière, d'augmenter la rigidité de la plaque. Les nervures 1604 sont des extensions selon la direction Z de plaque. Par exemple, la plaque de rigidification 1602 peut être divisée en au moins deux zones planes 1606, 1608 décalées l'une de l'autre le long de la direction Z et reliées entre elles par les nervures 1604. Les zones planes 1606, 1608 permettent en outre de définir des empreintes pour loger les composants de la station de mesure. Par exemple, l'enrouleur 802 qui se logent dans l'empreinte circulaire 1610 de la figure 16. L'épaisseur de la plaque de rigidification 1602 (avec les nervures) peut être comprise entre 5mm et 15mm, par exemple 10mm. L'épaisseur du matériau peut être compris entre 0,5mm et 2mm, par exemple 1mm.

. La géométrie des nervures et donc des zones planes 1606, 1608 dépend d'au moins deux facteurs : le facteur des contraintes mécaniques et le facteur de l'encombrement. Les nervures peuvent être arrangées en quadrillage, en maximisant les portions verticales (en Z) qui sont résistantes à la flexion, tout en laissant suffisamment de place en Z pour loger les composants

### (notamment l'enrouleur 802)

. Le cadre 1508 peut être monobloc ou être formé lui-même de plusieurs pièces. Il a une fonction esthétique (en tant que boitier qui cache les composants internes de la station de mesure 100) et peut avoir une fonction de solidarisation des pièces. Le cadre 1508 est par exemple en plastique (matière légère). Comme illustré sur les figures 15 et 17, le cadre 1508 comprend un fond 1510 (s'étendant essentiellement dans le plan XY) et une paroi latérale 1702 (s'étendant essentiellement selon la direction Z depuis le bord du cadre 1508). La patte 1504 peut s'étendre depuis le cadre 1508 (le fond 1510 en particulier). La patte 1504 passe alors par un trou 1614 correspondant de la plaque de rigidification 1602. Les orifices d'attaches 1502 peuvent aussi être monté sur le cadre 1508, en étant montés par exemple sur un écarteur 1512 qui s'étend selon la direction Z depuis le fond 1510. L'écarteur 1512 passe par un trou 1616 de la plaque de rigidification pour venir au contact de l'îlot 808 pour l'assemblage.

. Les éléments 1514 du cadre 1508 sont des plots de soudure par température (illustré en position fondue). En pratique, ils prennent la forme d'une tige en plastique qui s'insère dans un trou traversant 1612 correspondant de la plaque de rigidification 1602, puis un opérateur ou une machine vient faire fondre l'extrémité de la tige qui s'étale et vient maintenir ensemble la plaque de rigidification 1602 et le cadre 1508 (bouterollage). Alternativement, des vis peuvent être utilisées.

. Comme visible en figure 2, le bord de la plaque de support 202 est en retrait en X et en Y par rapport au celui du socle 108. Par conséquent, l'intérieur de la station de support 100 pourrait être visible. Pour éviter cela, la paroi latérale 1702 peut comprendre une collerette 1704, s'étendant dans un plan parallèle au plan XY depuis une extrémité de la paroi latérale 1702 vers l'intérieur du socle 108. Pour contribuer à tenir la plaque de rigidification 1602, la paroi latérale 1702 peut comprendre une pluralité de clips 1706 qui viennent coincer (par clipsage) la plaque de rigidification 1602.

. Dans une variante, l'écarteur 1512 est une pièce indépendante coincée entre le cadre 1508 (le fond 1510 en particulier) et la plaque de rigidification 1602. Cela permet de fabriquer le cadre 1508 en monobloc par injection en permettant le déplacement des injecteurs (sinon l'écarteur bloque leur passage).

. Chaque plot 1514 est un plot de soudage thermique. Il s'agit d'une pièce en plastique que l'on vient faire fondre pour qu'elle change de forme et bloque la pièce enfichée sur elle.

. Le cadre 1508 peut comprendre le chanfrein 204 qui facilite la préhension de la station de mesure 100. Le chanfrein 204 est présent entre le fond 1510 et la paroi latérale 1702 et autorise le passage de doigts.

. Le circuit électronique des cellules de charge peut être celui décrit dans les demandes PCT/FR2013/051754 ou bien PCT/FR2021/050661.

. Comme mentionné précédemment, la station de mesure 100, et plus particulièrement la base 102, peut comprendre un affichage 114 configuré pour afficher des informations à destination de l'utilisateur. L'affichage 114 est attaché à la plaque de support 202. La plaque de mesure 106 est positionnée au-dessus de l'affichage 114. Dans un mode de réalisation illustré en figures 8 et 9, la plaque de support 202 comprend un logement 810 apte à recevoir l'affichage 114. Afin que l'affichage 114 soit invisible lorsqu'il n'est pas alimenté et visible lorsqu'il est alimenté, la plaque de mesure 106 est traité différemment entre une zone au regard de l'affichage 114 et le reste de la plaque de mesure 106.

. Dans un mode de réalisation, les capteurs 104 incluent des chemins conducteurs d'électricité 602 (appelés « électrodes ») sur la base 102 (voir notamment figures 6 et 7). Les électrodes 602 peuvent prendre la forme d'un dépôt métallique sur une face supérieure 1104 de la plaque de mesure 106. La face supérieure 1104 de la plaque de mesure 106 est définie comme la face recevant les pieds de l'utilisateur (la face visible). Pour assurer la connexion électrique avec le PCB, les électrodes 602 passent par un bord de la plaque de mesure 106 et s'étendent jusque sur une face inférieure 1204 de la plaque de mesure 106. Le bord de plaque de mesure 106 peut présenter une forme arrondie pour assurer que le dépôt métallique se fasse bien et que la continuité électrique soit assurée. De plus, un bord arrondi permet d'éviter les risques de blessure lors de la préhension de la station de mesure 100. Par arrondi, il est signifié un arc de cercle ou des formes similaires. Le bord arrondi permet aussi de simplifier le dépôt métallique lors de la fabrication. La demande FR2106653, décrit en détail ces chemins conducteurs d'électricité.

. Les électrodes 602 sont connectées au PCB via un connecteur électrique, qui permet de faire la connexion entre le chemin conducteur d'électricité sur la face inférieure 1204 et le PCB monté sur la plaque de support 202. Le commutateur 566 permet de connecter et déconnecter les électrodes aux différents systèmes (système d'acquisition ECG, système d'impédancemétrie, système d'ESC, etc.). De la sorte, chaque électrode peut avoir plusieurs fonctions différentes en fonction de la position de commutation du commutateur 566. Le commutateur 566 comprend par exemple une pluralité d'interrupteurs pilotés par le MCU.

. La face supérieure 1104 de la base 102 comprend un groupe gauche LG d'électrodes (destiné à être en contact avec le pied gauche, et un groupe droit RG d'électrodes destiné à être en contact avec le pied droit. Lorsque la base 102 est posée en condition normale d'utilisation, l'utilisateur met ses pieds sur une partie gauche de la balance et une partie droite de la balance (les doigts de pied étant du côté de l'affichage 114). Les figures 10 et 11 représentent des chemins conducteurs d'électricité L1, L3, L5, L7, L9, L11, L13, L15, L17 qui forment les électrodes du groupe gauche LG d'électrodes et des chemins conducteurs d'électricité R2, R4, R6, R8, R10, R12, R14, R16, R18, qui forment les électrodes du groupe droite RG d'électrodes.

. Les électrodes de la base 102 peuvent prendre la forme de bandes parallèles les unes aux autres le long de la direction X (le long de la largeur de la base 102).

. Dans l'architecture illustrée, les couples de chemins L1 et L3 ; L15 et L17 ; R2 et R4 ; R16 et R18 ne sont pas indépendants mais sont reliés électriquement en permanence, de sorte que la base 102 comprend en pratique sept électrodes indépendantes dans le groupe gauche LG et sept électrodes indépendantes dans le groupe droit RG. Ces connexions électriques permanentes peuvent se faire via les chemins électriques sur la plaque de mesure 106 (par exemple sur la face inférieure 1204, non-illustré) ou via le PCB de la station de mesure 100.

. Dans un exemple, les chemins conducteurs d'électricité de la face supérieure 1104 correspondant aux électrodes 1301-1312 ont une dimension (sur la face supérieure 1104) selon la longueur Y comprise entre 1,5cm et 2 cm (par exemple 1,7cm) ; l'espacement entre deux bandes successives peut être compris entre 0,5cm et 1cm (par exemple 0,85cm) ; les électrodes peuvent avoir une dimension selon la largeur X supérieure à 10cm.En particulier, chaque groupe LG, RG peut comprendre au moins quatre électrodes indépendantes pour notamment pouvoir effectuer un IPG dans le pied (deux électrodes connectées à la source de courant alternatif 560 et deux électrodes connectées au voltmètre 562). Dans un autre mode de réalisation, chaque groupe LR, RG peut comprendre au moins deux électrodes indépendantes (pour effectuer un ESC avec anode/cathode et une électrode haute impédance, ou pour effectuer un BIA ou un IPG entre les jambes), ou trois électrodes indépendantes.

## Revendications

1. Station de mesure (100) comprenant :
- une base (102) comprenant :
∘ une plaque de mesure (106) présentant une face supérieure (1104) apte à recevoir les pieds d'un utilisateur,
∘ un support de poignée (112), monté sur la face supérieure (1104) de la plaque de mesure (106), le support de poignée (112) formant un berceau (1032) configuré pour recevoir la poignée (110),
- une poignée (110) apte à recevoir les mains d'un utilisateur, la poignée (110) étant configurée pour être reçue sur le support de poignée (112),
**caractérisée en ce que** le berceau s'étend sur une surface, dans une section transversale du support de poignée (112), de moins de 90° de part et d'autre à partir du fond du berceau.

2. Station de mesure (100) selon la revendication 1, comprenant un câble (302) reliant la poignée (110) à la base (102) et dans laquelle la plaque de mesure (106) comprend un orifice traversant (1102) au travers duquel passe le câble (302) reliant la poignée (110) à la base (102).

3. Station de mesure (100) selon la revendication 2, dans laquelle le support de poignée (112) comprend un orifice traversant (1304) au travers duquel passe le câble (302).

4. Station de mesure (100) selon la revendication 3, dans laquelle l'orifice traversant (1304) du support de poignée (112) est positionné au fond du berceau (1302).

5. Station de mesure (100) selon la revendication 3 ou 4, dans lequel les deux orifices traversants (1102, 1304) sont en regard l'un de l'autre.

6. Station de mesure (100) selon la revendication 5, dans laquelle le câble (302) s'étend orthogonalement depuis une surface de la poignée, afin de pouvoir s'insérer sans changement de direction dans les orifices traversants lorsque la poignée (110) est positionnée dans le support de poignée (112).

7. Station de mesure (100) selon l'une quelconque des revendications 2 à 6, dans laquelle la base (102) comprend un enrouleur (802) apte à enrouler et dérouler le câble (302) et la base comprend un rouleau (1002) configuré pour faciliter un changement de direction du câble (302) lors de l'enroulement et du déroulement du câble (802).

8. Station de mesure (100) selon la revendication 7 comprenant un deuxième rouleau configuré pour canaliser le déplacement du câble, les deux rouleaux étant de part et d'autre du câble.

9. Station de mesure (100) selon l'une quelconque des revendications 2 à 9, dans laquelle la poignée (110) s'étend entre deux extrémités et comprend une pluralité d'électrodes (602, LH1, LH2, RH2, RH1) disposées à la suite les unes des autres entre les deux extrémités, dans laquelle les électrodes s'étendent, dans une section orthogonale, au plus sur une surface externe de la poignée comprise entre -45° et +90°, 0° étant défini par une position d'insertion du câble dans la poignée (110), les angles positifs étant définis à partir de 0°en allant du côté d'un bord antérieur (206) de la station de mesure et les angles négatifs étant définis à partir de 0° en allant du côté d'un bord postérieur (208) de la station de mesure.

10. Station de mesure (100) selon la revendication 9, dans laquelle le berceau s'étend sur une surface dont l'angle dans une section transversale du support de poignée, est au moins égal à celui des électrodes dans le sens des angles négatifs, lorsque la poignée est en position rangée.

11. Station de mesure (100) selon l'une quelconque des revendications 1 à 10, dans laquelle le berceau (1302) a une forme complémentaire à celle de la poignée (110).

12. Station de mesure (100) selon l'une quelconque des revendications 1 à 11, dans laquelle la plaque de mesure (106) est plane.

13. Station de mesure (100) selon l'une quelconque des revendications 1 à 12, dans laquelle la plaque de mesure (106) est en verre.

14. Station de mesure (100) selon l'une quelconque des revendications 1 à 13, dans laquelle la poignée (110) est configurée pour permettre l'acquisition d'un électrocardiogramme (ECG) et une analyse d'impédance (BIA, IPG).

15. Station de mesure (100) selon l'une quelconque des revendications 1 à 14, dans laquelle la poignée est surélevée en position rangée par rapport à la plaque de mesure (106).

## Patentansprüche

1. Messstation (100), bestehend aus :
- eine Basis (102) mit:
∘ eine Messplatte (106) mit einer Oberseite (1104), die geeignet ist, die Füße eines Benutzers aufzunehmen,
∘ einen Griffhalter (112), der an der Oberseite (1104) der Messplatte (106) angebracht ist, wobei der Griffhalter (112) eine Wiege (1032) bildet, die so konfiguriert ist, dass sie den Griff (110) aufnimmt,
- einen Griff (110), der geeignet ist, die Hände eines Benutzers aufzunehmen, wobei der Griff (110) so konfiguriert ist, dass er auf der Griffhalterung (112) aufgenommen werden kann,
**dadurch gekennzeichnet, dass** sich die Wiege über eine Fläche in einem Querschnitt des Griffträgers (112) von weniger als 90° auf jeder Seite vom Boden der Wiege aus erstreckt.

2. Messstation (100) nach Anspruch 1, die ein Kabel (302) umfasst, das den Griff (110) mit der Basis (102) verbindet, und wobei die Messplatte (106) eine Durchgangsöffnung (1102) umfasst, durch die das Kabel (302) verläuft, das den Griff (110) mit der Basis (102) verbindet.

3. Messstation (100) nach Anspruch 2, wobei der Griffhalter (112) eine Durchgangsöffnung (1304) aufweist, durch die das Kabel (302) verläuft.

4. Messstation (100) nach Anspruch 3, wobei die Durchgangsöffnung (1304) des Griffhalters (112) am Boden der Wiege (1302) positioniert ist.

5. Messstation (100) nach Anspruch 3 oder 4, wobei die beiden Durchgangsöffnungen (1102, 1304) einander zugewandt sind.

6. Messstation (100) nach Anspruch 5, wobei sich das Kabel (302) orthogonal von einer Oberfläche des Griffs erstreckt, so dass es ohne Richtungsänderung in die Durchgangslöcher einführbar ist, wenn der Griff (110) in der Griffhalterung (112) positioniert ist.

7. Messstation (100) nach einem der Ansprüche 2 bis 6, wobei die Basis (102) eine Aufwickelvorrichtung (802) umfasst, die zum Aufwickeln und Abwickeln des Kabels (302) geeignet ist, und die Basis eine Rolle (1002) umfasst, die so konfiguriert ist, dass sie eine Richtungsänderung des Kabels (302) beim Aufwickeln und Abwickeln des Kabels (802) erleichtert.

8. Messstation (100) nach Anspruch 7 mit einer zweiten Rolle, die so konfiguriert ist, dass sie die Bewegung des Kabels kanalisiert, wobei sich die beiden Rollen auf beiden Seiten des Kabels befinden.

9. Messstation (100) nach einem der Ansprüche 2 bis 9, wobei sich der Griff (110) zwischen zwei Enden erstreckt und eine Vielzahl von Elektroden (602, LH1, LH2, RH2, RH1) umfasst, die aufeinanderfolgend zwischen den beiden Enden angeordnet sind, wobei sich die Elektroden in einem orthogonalen Querschnitt erstrecken, höchstens über eine Außenfläche des Griffs zwischen -45° und +90° erstrecken, wobei 0° durch eine Einführposition des Kabels in den Griff (110) definiert ist, wobei positive Winkel ab 0° von der Seite einer Vorderkante (206) der Messstation aus definiert sind und negative Winkel ab 0° von der Seite einer Hinterkante (208) der Messstation aus definiert sind.

10. Messstation (100) nach Anspruch 9, wobei sich die Wiege über eine Fläche erstreckt, deren Winkel in einem Querschnitt des Griffträgers mindestens gleich dem der Elektroden in Richtung negativer Winkel ist, wenn sich der Griff in der verstauten Position befindet.

11. Messstation (100) nach einem der Ansprüche 1 bis 10, wobei die Wiege (1302) eine Form hat, die komplementär zu der des Griffs (110) ist.

12. Messstation (100) nach einem der Ansprüche 1 bis 11, wobei die Messplatte (106) eben ist.

13. Messstation (100) nach einem der Ansprüche 1 bis 12, wobei die Messplatte (106) aus Glas besteht.

14. Messstation (100) nach einem der Ansprüche 1 bis 13, wobei der Griff (110) so konfiguriert ist, dass er die Erfassung eines Elektrokardiogramms (EKG) und einer Impedanzanalyse (BIA, IPG) ermöglicht.

15. Messstation (100) nach einem der Ansprüche 1 bis 14, wobei der Griff in der aufgereihten Position in Bezug auf die Messplatte (106) erhöht ist.

## Claims

1. A measuring station (100) comprising:
- a base (102) comprising:
∘ a measurement plate (106) with an upper surface (1104) adapted to receive a user's feet,
∘ a handle support (112), mounted on the upper face (1104) of the measurement plate (106), the handle support (112) forming a cradle (1032) configured to receive the handle (110),
- a handle (110) adapted to receive a user's hands, the handle (110) being configured to be received on the handle support (112),
**characterised in that** the cradle extends over an area, in a cross-section of the handle support (112), of less than 90° on either side from the bottom of the cradle.

2. The measurement station (100) according to claim 1, comprising a cable (302) connecting the handle (110) to the base (102) and wherein the measurement plate (106) comprises a through hole (1102) through which the cable (302) connecting the handle (110) to the base (102) passes.

3. The measurement station (100) according to claim 2, wherein the handle support (112) comprises a through hole (1304) through which the cable (302) passes.

4. The measurement station (100) according to claim 3, wherein the through hole (1304) of the handle support (112) is positioned at the bottom of the cradle (1302).

5. The measurement station (100) according to claim 3 or 4, in which the two through orifices (1102, 1304) face each other.

6. The measurement station (100) according to claim 5, wherein the cable (302) extends orthogonally from a surface of the handle, so as to be insertable without change of direction into the through-holes when the handle (110) is positioned in the handle support (112).

7. The measurement station (100) according to any one of claims 2 to 6, wherein the base (102) comprises a reel (802) capable of winding and unwinding the cable (302) and the base comprises a roller (1002) configured to facilitate a change in direction of the cable (302) during winding and unwinding of the cable (802).

8. The measurement station (100) according to claim 7 comprising a second roller configured to channel the movement of the cable, the two rollers being on either side of the cable.

9. The measurement station (100) according to any one of claims 2 to 9, wherein the handle (110) extends between two ends and comprises a plurality of electrodes (602, LH1, LH2, RH2, RH1) arranged in succession between the two ends, wherein the electrodes extend, in an orthogonal section at most on an external surface of the handle between -45° and +90°, 0° being defined by a position of insertion of the cable in the handle (110), the positive angles being defined from 0° going to the side of a front edge (206) of the measurement station and the negative angles being defined from 0° going to the side of a rear edge (208) of the measurement station.

10. The measurement station (100) according to claim 9, wherein the cradle extends over a surface whose angle in a cross-section of the handle support is at least equal to that of the electrodes in the direction of the negative angles, when the handle is in the stowed position.

11. The measurement station (100) according to any one of claims 1 to 10, in which the cradle (1302) has a shape complementary to that of the handle (110).

12. The measurement station (100) according to any one of claims 1 to 11, in which the measurement plate (106) is flat.

13. The measurement station (100) according to any one of claims 1 to 12, in which the measurement plate (106) is made of glass.

14. The measurement station (100) according to any one of claims 1 to 13, in which the handle (110) is configured to allow acquisition of an electrocardiogram (ECG) and an impedance analysis (BIA, IPG).

15. The measurement station (100) according to any one of claims 1 to 14, in which the handle is raised in a stowed position relative to the measurement plate (106).
